# EUROPEAN PATENT APPLICATION

(11) **EP 4 563 073 A1**
(43) Date of publication of application: **04.06.2025**
(21) Application number: 22957018.9
(22) Date of filing: 02.09.2022
(51) Int. Cl.: A61B 5/00

(54) **MOBILE MONITORING DEVICE, MONITORING SYSTEM, AND METHOD FOR COLLECTING AND TRANSMITTING PHYSIOLOGICAL PARAMETER DATA OF WEARER**

(71) Applicant: Shenzhen Mindray Bio-Medical Electronics Co., Ltd., Shenzhen, Guangdong 518057 (CN)
(72) Inventor: XIA, Hengxing, Shenzhen, Guangdong 518057 (CN); ZHANG, Kui, Shenzhen, Guangdong 518057 (CN); LIU, Qiling, Shenzhen, Guangdong 518057 (CN); XU, Li, Shenzhen, Guangdong 518057 (CN)
(74) Representative: KIPA AB
(86) International application number: PCT/CN2022/116916
(87) International publication number: WO 2024/045199

(57) **Abstract**

Provided are a mobile monitoring device, a monitoring system, and a method for collecting and transmitting physiological parameter data of a wearer. The mobile monitoring device comprises: at least one group of monitoring hosts, each monitoring host comprising a communication module and a power supply module; and an accessory portion, the monitoring host being detachably coupled to the accessory portion and communicating with the accessory portion. The accessory portion comprises: a wearing portion and a first memory. The mobile monitoring device is removably fixed to a specific body part of the wearer by means of the wearing portion. The first memory stores first configuration information used for carrying out first configuration for the mobile monitoring device. When the monitoring host is coupled to the accessory portion, the monitoring host is configured to acquire at least part of the first configuration information from the first memory by means of a second electric connection portion and carry out corresponding configuration. According to the mobile monitoring device, the monitoring system, and the method for collecting and transmitting the physiological parameter data of the wearer of the present application, configuration can be automatically carried out when the monitoring host is replaced, thereby achieving high usability.

## Description

### TECHNICAL FIELD

The disclosure relates to a technical field of a monitoring device, and in particular to a mobile monitoring device, a monitoring system, and a method for collecting and transmitting physiological parameter data of a wearer.

### BACKGROUND

Currently, a mobile monitoring device (such as a medical-grade wearable wireless sensor device) usually has a built-in battery, and achieves goals of smaller size and lighter weight through a more compact design of an internal structure. When power of the battery is low, the mobile monitoring device needs to be removed from a wearable part of a wearer and then charged.

Charging time of the mobile monitoring device is long, but a monitoring for a vital sign of a patient cannot be interrupted. Therefore, after a mobile monitoring device with low power is decoupled from a body of a user, another mobile monitoring device with sufficient power needs to be used as soon as possible to continue monitoring the vital sign of the patient. However, the replaced mobile monitoring device requires the staff to manually reconfigure it, which is less usable.

Therefore, an improvement is needed to at least partially solve an above problem.

### SUMMARY

A series of simplified concepts are introduced into summary of this disclosure, and are further elaborated in specific embodiments of this disclosure. The summary of this disclosure does not attempt to define key and necessary technical characteristics of the claimed technical solution, nor attempt to determine a protection scope of the claimed technical solution.

According to a first aspect of this disclosure, a mobile monitoring device is provided, wherein the mobile monitoring device is fixed to a wearer, which fixation is capable of being unfixed, wherein the mobile monitoring device is configured to collect physiological parameter data of the wearer; wherein the mobile monitoring device is in communicative connection with a target monitoring device, so as to transmit the collected physiological parameter data to the target monitoring device; wherein the mobile monitoring device includes:
at least one monitoring host, wherein the monitoring host includes a communication module and a power supply module; wherein the mobile monitoring device is in communicative connection with the target monitoring device through the communication module; and
an accessory part, wherein the monitoring host is coupled to the accessory part, which coupling is capable of being decoupled, and the monitoring host communicates with the accessory part; wherein the accessory part is provided with a first electrical connection part, the monitoring host is provided with a second electrical connection part, the monitoring host and the accessory part communicate with each other through the first electrical connection part and the second electrical connection part;
wherein the accessory part includes a wearable part and a first memory; the mobile monitoring device is fixed to a designated body part of the wearer through the wearable part, which fixation is capable of being unfixed, the first memory is configured to store first configuration information for performing first configuration on the mobile monitoring device, wherein when the monitoring host is coupled to the accessory part, the monitoring host is configured to obtain, from the first memory, at least a portion of the first configuration information through the second electrical connection part, and perform the first configuration.

According to a second aspect of this disclosure, a method for collecting and transmitting physiological parameter data of a wearer is provided, which method is applicable to a mobile monitoring device and includes:
collecting the physiological parameter data of the wearer through a sensor which is connected with a test site of the wearer;
transmitting the collected physiological parameter data to an accessory part which is connected with the sensor;
transmitting, through the accessory part, the collected physiological parameter data to a monitoring host; wherein the monitoring host is coupled to the accessory part, which coupling is capable of being decoupled, and the monitoring host communicates with the accessory part, the accessory part includes a wearable part which is fixed to a designated body part of the wearer, which fixation is capable of being unfixed; and
transmitting, through the monitoring host, the collected physiological parameter data to a target monitoring device; and/or processing at least a portion of the collected physiological parameter data and transmitting a processed result to a target monitoring device;
wherein the method further includes:
   obtaining, by the monitoring host, first configuration information for configuring the mobile monitoring device, from a first memory which is arranged at the accessory part, when the monitoring host is coupled to the accessory part.

According to a third aspect of this disclosure, a method for collecting and transmitting physiological parameter data of a wearer is provided, which method is applicable to a mobile monitoring device; wherein the mobile monitoring device is fixed to the wearer, which fixation is capable of being unfixed; wherein the mobile monitoring device is configured to collect physiological parameter data of the wearer; wherein the mobile monitoring device is in communicative connection with a target monitoring device, so as to transmit the collected physiological parameter data to the target monitoring device; wherein the mobile monitoring device includes a monitoring host and an accessory part; the monitoring host is coupled to the accessory part, which coupling is capable of being decoupled, and the monitoring host communicates with the accessory part; the accessory part includes a wearable part and a first memory; the mobile monitoring device is fixed to a designated body part of the wearer through the wearable part, which fixation is capable of being unfixed; wherein the mobile monitoring device includes a first monitoring host and a second monitoring host, wherein the method includes:
obtaining configuration information for wireless communication, which information is required for performing a pairing operation between the first monitoring host and the target monitoring device, when the first monitoring host is coupled to the accessory part;
performing the pairing operation between the first monitoring host and the target monitoring device, based on the configuration information for wireless communication;
establishing a first wireless communicative connection between the first monitoring host and the target monitoring device through a wireless communication module of the first monitoring host, after the first monitoring host and the target monitoring device have successfully paired with each other;
outputting to the first memory for storing first configuration information which includes the configuration information for wireless communication;
controlling the second monitoring host to obtain, from the first memory, the configuration information for wireless communication, when the first monitoring host is decoupled from the accessory part and the second monitoring host is coupled to the accessory part;
performing a pairing operation between the second monitoring host and the target monitoring device, based on the configuration information for wireless communication; and
establishing a second wireless communicative connection between the second monitoring host and the target monitoring device through a wireless communication module of the second monitoring host, after the second monitoring host and the target monitoring device have successfully paired with each other.

According to a fourth aspect of this disclosure, a mobile monitoring device is provided, including:
a monitoring host, which includes a power supply module, wherein the power supply module is assembled at the monitoring host, which assembly is capable of being disassembled;
an accessory part, wherein the monitoring host is coupled to the accessory part, which coupling is capable of being decoupled, and the monitoring host communicates with the accessory part; wherein the accessory part is provided with a first electrical connection part, the monitoring host is provided with a second electrical connection part, the monitoring host and the accessory part communicate with each other through the first electrical connection part and the second electrical connection part; wherein the accessory part includes a wearable part, a sensor, and a first memory; the mobile monitoring device is fixed to a designated body part of the wearer through the wearable part, which fixation is capable of being unfixed, the sensor is connected with a test site of the wearer so as to collect physiological parameter data; the first memory is configured to store first configuration information for performing first configuration on the mobile monitoring device, wherein when the monitoring host is coupled to the accessory part, the monitoring host is configured to obtain, from the first memory, at least a portion of the first configuration information through the second electrical connection part, and perform the first configuration;
a communication module, through which the mobile monitoring device and the target monitoring device are in communicative connection with each other, wherein the communication module is arranged at the monitoring host or the accessory part.

According to a fifth aspect of this disclosure, a monitoring system is provided, including:
the aforementioned mobile monitoring device, which is configured to obtain the physiological parameter data, and transmit to the target monitoring device, real-time data and historical data of the physiological parameter data which is obtained by the mobile monitoring device, when the mobile monitoring device and the target monitoring device are in communicative connection with each other;
the target monitoring device which is in communicative connection with the mobile monitoring device and configured to obtain and display the physiological parameter data.

According to the mobile monitoring device, the monitoring system and the method for collecting and transmitting physiological parameter data of a wearer, according to this disclosure, the monitoring host and the accessory part are in a connection which is capable of being disconnected, such that the monitoring host with low power can be replaced without removing the accessory part from the patient, which significantly reduces an intrusion to the user. Furthermore, the first memory arranged at the accessory part enables the replaced monitoring host to obtain configuration information therefrom and automatically configure said monitoring host itself without the need for the user to manually reconfigure monitoring host, thereby significantly improving ease of use.

### BRIEF DESCRIPTION OF THE DRAWINGS

The following drawings of this disclosure are incorporated herein as part of this disclosure and are useful for understanding this disclosure. The accompanying drawings show embodiments of this disclosure and their descriptions, which are used to explain apparatus and principles of this disclosure.

In the accompanying drawings:
FIG. 1 is a structural block diagram of a mobile monitoring device according to an embodiment of this disclosure.
FIG. 2 is a three-dimensional diagram of a mobile monitoring device according to an embodiment of this disclosure.
FIG. 3 is an exploded diagram of the mobile monitoring device in FIG. 2.
FIG. 4 is a three-dimensional diagram of a monitoring host of the mobile monitoring device in FIG. 2.
FIG. 5 is an exploded diagram of the monitoring host in FIG. 4.
FIG. 6 is a perspective diagram of a base and a connector of the mobile monitoring device in FIG. 2.
FIG. 7 is an exploded diagram of a base and a connector of the mobile monitoring device in FIG. 6.
FIG. 8 is a cross-sectional diagram of the base along line A-A in FIG. 6.
FIG. 9 is an exploded diagram of a mobile monitoring device according to another embodiment of this disclosure.
FIG. 10 is a perspective diagram of a base and a connector according to another embodiment of this disclosure.
FIG. 11 is a three-dimensional diagram of a base and a connector according to a further embodiment of this disclosure.
FIG. 12 is a three-dimensional diagram of a mobile monitoring device according to a further embodiment of this disclosure.
FIG. 13 is a flow chart of a method for collecting and transmitting physiological parameter data of a wearer according to an embodiment of this disclosure.
FIG. 14 is a flow chart of a method for collecting and transmitting physiological parameter data of a wearer according to an embodiment of this disclosure.
FIG. 15 is a structural block diagram of a mobile monitoring device according to an embodiment of this disclosure.
FIG. 16 is a structural block diagram of a mobile monitoring system according to an embodiment of this disclosure.
FIG. 17 is a flow chart of a method for wireless pairing of a mobile monitoring device according to an embodiment of this disclosure.
FIG. 18 is a structural block diagram of a monitoring system according to an embodiment of this disclosure.
FIG. 19 is a diagram of a monitoring system according to an embodiment of this disclosure.
FIG. 20 is a diagram of a single-patient usage scenario for the mobile medical monitoring system in FIG. 19.
FIG. 21 is a diagram of a multi-patient usage scenario for the mobile medical monitoring system in FIG. 19.

### DESCRIPTION OF THE PREFERRED EMBODIMENTS

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

It will be understood that although terms, first, second, third, etc. may be used to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be described as a second element, component, region, layer or section without departing from the teachings of this disclosure.

The purpose of the terminology used here is only to describe specific embodiments and is not a limitation of this disclosure. When used herein, the singular forms of "an", "a", and "the/said" are also intended to include the plural form, unless the context clearly indicates another way. It should also be understood that the terms, such as "including", "and/or", "consisting of", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items.

Various embodiments of this disclosure are described below with reference to the accompanying drawings, wherein the technical features of the various embodiments may be combined with each other without conflict.

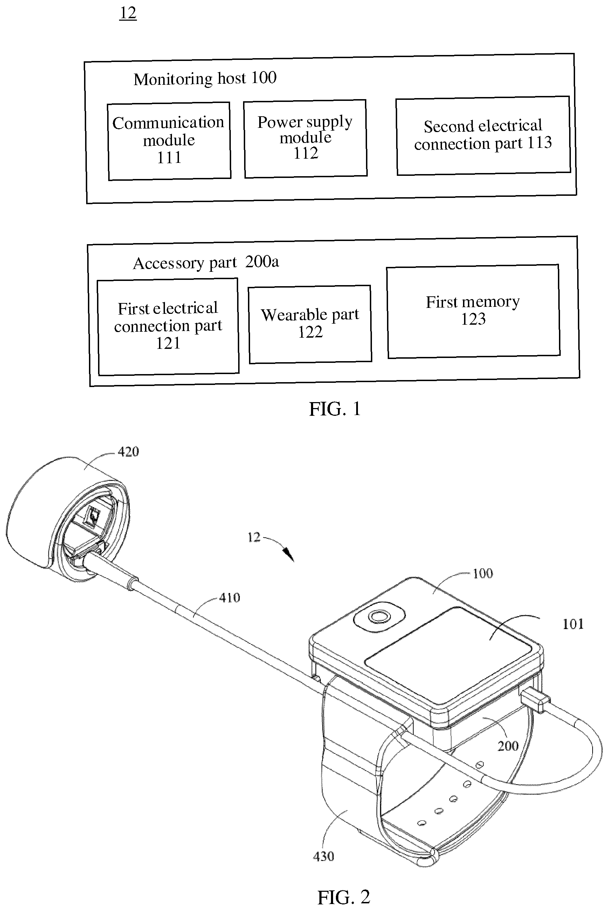

First, referring to FIG. 1, a mobile monitoring device 12 according to an embodiment of this disclosure is exemplarily described. The mobile monitoring device 12 is fixed to a wearer, which fixation is capable of being unfixed; wherein the mobile monitoring device is configured to collect physiological parameter data of the wearer. The mobile monitoring device 12 is in communicative connection with a target monitoring device and transmits the collected physiological parameter data to the target monitoring device. The target monitoring device may be one or more of: a bedside monitor, a central station, a department-level workstation device, and a hospital-level data center/a management device of a hospital-level emergency center. The mobile monitoring device 12 includes a monitoring host 100 and an accessory part 200 a.

The monitoring host 100 includes a communication module 111 and a power supply module 112. The mobile monitoring device 12 is in communicative connection with a target monitoring device through the communication module 111 so as to transmit to the target monitoring device the physiological parameter data which is collected by the mobile monitoring device 12. The communication module 111 may be a wireless communication module, which may include at least one of a Bluetooth module, a WMTS (Wireless Medical Telemetry Services) communication module, a contactless (full-range) communication module, and a WIFI communication module. The power supply module 112 may include a rechargeable battery, which may be used to supply power to components which are inside the mobile monitoring device 12 and require power.

The monitoring host 100 is coupled to the accessory part 200a and communicates with the accessory part 200a, which coupling is capable of being decoupled. Therefore, while the monitoring host 100 realizes a monitoring function, it is also convenient for medical staff to remove the monitoring host 100 when necessary and perform an operation, such as charging, replacing batteries, cleaning and disinfection. The accessory part 200 a is provided with a first electrical connection part 121, and the monitoring host 100 is provided with a second electrical connection part 113. The monitoring host 100 and the accessory part 200 a communicate with each other through the first electrical connection part 121 and the second electrical connection part 113. That is, the monitoring host 100 and the accessory part 200a communicate with each other through a physical connection.

The monitoring host 100 includes a housing and a first snap-fit part and a second electrical connection part which are arranged at the housing, wherein the second electrical connection part is a conductive contact. The housing surrounds an internal space of the monitoring host, in which a first circuit board is arranged, and the conductive contact is electrically connected with the circuit board. The accessory part includes a bottom housing, a second snap-fit part and a connector receiving part, which are arranged at the bottom housing, wherein a connector is arranged in the connector receiving part. A position of the second snap-fit part at the bottom housing corresponds a position of the first snap-fit part at the monitoring host, thereby achieving a connection between the monitoring host and the accessory part, which connection is capable of being disconnected. The connector includes a housing, a second circuit board and a first electrical connection part which are arranged inside the housing. The first electrical connection part is a conductive pogo pin, which can be arranged on the second circuit board and corresponds to a conductive contact. When the first snap-fit part and the second snap-fit part are snap-fitted, the conductive contact and the conductive pogo pin are in contact to achieve an electrical connection.

The accessory part 200a further includes a wearable part 122 and a first memory 123. Optionally, the wearable part 122 can be implemented as a base, which can be used in conjunction with a wearable component, such as a wristband, a clip and another structure, and is fixed to a designated body part of a user (such as a wrist, arm, etc.) which fixation is capable of being unfixed, so that when the monitoring host 100 is coupled to the accessory part 200a, the mobile monitoring device 12 can be fixed to the designated body part of the wearer through the wearable part 122, which fixation is capable of being unfixed. Since the monitoring host 100 and the accessory part 200a are coupled with each other, which coupling is capable of being decoupled; when the monitoring host 100 is low on power, medical staff can remove the monitoring host 100 separately for replacement without removing the wearable part 122 from the body of the wearer, thereby reducing intrusion to the wearer. The first memory 123 is configured to store first configuration information for performing first configuration on the mobile monitoring device 12. When the monitoring host 100 is coupled to the accessory part 200a, the monitoring host 100 is configured to obtain, from the first memory, at least a portion of the first configuration information 123 through the second electrical connection part 113, and perform the first configuration. Thus, when the monitoring host 100 is low on power and the medical staff replaces the monitoring host 100, the newly replaced monitoring host 100 can automatically obtain, from the first memory, at least a portion of the first configuration information 123 and perform the first configuration after being coupled to the accessory part 200a, without the need for the medical staff to manually configure the monitoring host 100.

In an embodiment of this disclosure, the first configuration information may include at least one of: configuration information for wireless communication, wearer information, and configuration information for the monitoring host. The configuration information for wireless communication may include at least one of: a device identification code, a device SN (serial number) code, a randomly generated pairing code, a frequency, a communication channel, a baud rate, an MAC address, an IP address, a service set identifier (referred to as SSID), a WEP (Wired Equivalent Privacy) protocol, a WPA (Wi-Fi Protected Access) protocol, a WPA2 protocol, a WPA3 protocol, an EAP (Extensible Authentication Protocol), and other protocol-related information, and a communication port. The wearer information includes at least one of: a gender, a classification, an age, a height, a weight, a bed number, a department, and a medical history. The configuration information for the monitoring host may include at least one of: a sensor type, a configuration parameter for analysis algorithm, and a configuration parameter for working mode. In some embodiments, after a newly replaced monitoring host 100 obtains at least a portion of the first configuration information and performs corresponding configuration, the newly replaced monitoring host 100 can continue the work performed by the monitoring host 100 before replacement, thereby ensuring a continuity of the monitoring process and a consistency of the monitoring result.

In an embodiment of this disclosure, when the monitoring host 100 is coupled to the accessory part 200a, the power supply module 112 is in electrical connection with the first memory 123, for example, in electrical connection with the first memory 123 through the second electrical connection part 113 and the first electrical connection part, thereby supplying power to the first memory 123. Accordingly, the first memory 123 may be a non-volatile memory. When the monitoring host 100 is replaced, even if the first memory 123 is temporarily powered off, the first configuration information stored therein will not be lost.

In an embodiment of this disclosure, the mobile monitoring device 12 further includes a sensor and a parameter cable. The sensor is connected with a test site of the wearer, so as to collect corresponding physiological parameter data. Specifically, the sensor can be one or more of: a blood oxygen probe (which includes a light emitting element and a light receiving element), an ECG electrode, a body temperature sensor, and a blood pressure sensor. Accordingly, the physiological parameter data collected by the sensor includes one or more of: a blood oxygen parameter, an ECG parameter, a body temperature parameter, and a blood pressure parameter. The parameter cable is a cable used for data transmission, one end of which is connected with the sensor and the other end of which is connected with the accessory part 200a, so as to transmit the physiological parameter data collected by the sensor to the accessory part 200a, and then to the monitoring host 100. In some embodiments, the parameter cable is connected with the sensor, which connection is not capable of being disconnected; for example, the parameter cable is fixedly connected with the sensor, by welding, gluing, etc. In some embodiments, the parameter cable is connected with the sensor, which connection is capable of being disconnected, for example, the parameter cable is connected with the sensor via a plug connector and a plug slot, which connection is capable of being disconnected. In some embodiments, the parameter cable is connected with the accessory part 200a, which connection is not capable of being disconnected; for example, the parameter cable is fixedly connected with the accessory part 200a, by welding, gluing, etc. In some embodiments, the parameter cable is connected with the accessory part 200a, which connection is capable of being disconnected, for example, the parameter cable is connected with the accessory part 200a via a plug connector and a plug slot, which connection is capable of being disconnected.

In an embodiment of this disclosure, the mobile monitoring device 12 further includes a processor. The processor is arranged at or near the monitoring host 100. The processor is in communicative connection with the sensor and the communication module 111. The processor is configured to control the communication module 111 to transmit the collected physiological parameter data to a target monitoring device, and/or to process at least a portion of the collected physiological parameter data and control the communication module 111 to transmit a processed result to a target monitoring device. In some embodiments, the processor may process ECG parameter data which is collected by the sensor, so as to obtain a heart rate value, waveform data, etc., and then control the communication module 111 to transmit the obtained heart rate value and waveform data to the target monitoring device. In some embodiments, the processor can determine whether arrhythmia occurs based on a preset rule and the collected ECG parameter data. When arrhythmia is determined to occur, an arrhythmia alarm event is generated, and then the communication module 111 is controlled to transmit the arrhythmia alarm event to the target monitoring device, so that medical staff can handle it in time. For other physiological parameter data except the ECG parameter data, the processor can process such data in a similar manner and control the communication module 111 to transmit the processed result to the target monitoring device, which is not described in detail here.

Optionally, the processor may be implemented as software, hardware, firmware, or any combination thereof, and may use a single or multiple application specific integrated circuits (ASICs), a single or multiple general purpose integrated circuits, a single or multiple microprocessors, a single or multiple programmable logic devices, or any combination of the foregoing circuits and/or devices, or other suitable circuits or devices.

In this disclosure, physiological parameter data may include at least one of: an electrocardiogram parameter value, a respiratory parameter value, a blood oxygen parameter value, a blood pressure parameter value, a body temperature parameter value, a sleep parameter value, an exercise parameter value, a pain parameter values, or other parameter data that needs to be monitored.

In an embodiment of this disclosure, the power supply module 112 is configured to be in electrical connection with the sensor so as to supply power to the sensor, when the monitoring host 100 is coupled to the accessory part 200a. In an embodiment of this disclosure, when the processor is arranged at the monitoring host 100, the power supply module 112 is further configured to be in electrical connection with the processor so as to supply power to the processor. When the processor is arranged at the monitoring host 100, the power supply module 112 is further configured to be in electrical connection with the processor so as to supply power to the processor, when the monitoring host 100 is coupled to the accessory part 200a.

In an embodiment of this disclosure, the first memory 123 can be arranged at any position of the accessory part 200a. In some embodiments, the first memory 123 may be arranged inside the wearable part 122. In some embodiments, the first memory 123 may be arranged inside a parameter cable or a sensor.

In an embodiment of this disclosure, the first configuration at least includes a pairing operation between the mobile monitoring device 12 and the target monitoring device. Specifically, while a mobile monitoring device 12 establishes a communicative connection with a target monitoring device for the first time, the processor is further configured to perform a pairing operation between the mobile monitoring device 12 and the target monitoring device. It should be noted that "a first time" here refers to a first time for a new patient; "mobile monitoring device 12" in the expression that "while a mobile monitoring device 12 establishes a communicative connection with a target monitoring device for the first time", refers to a mobile monitoring device 12 which is worn by the new patient for the first time, that is, a first monitoring host 100, which is coupled to an accessory part 200a which is worn on a new patient for the first time, as well as said accessory part 200a, together form said mobile monitoring device 12. In some embodiments, a process for the processor to perform the pairing operation between the mobile monitoring device 12 and the target monitoring device requires manual operation by medical staff, for example, the medical staff needs to manually input a pairing code on the mobile monitoring device 12 or the target monitoring device. After the mobile monitoring device 12 and the target monitoring device have successfully paired with each other, the processor is configured to establish a communicative connection between the mobile monitoring device 12 and the target monitoring device, obtain the first configuration information, and output the first configuration information to the first memory 123 for storing. The first configuration information at least includes configuration information for wireless communication, which information is required to perform the pairing operation between the mobile monitoring device 12 and the target monitoring device. Therefore, when the monitoring host is subsequently replaced, the newly replaced monitoring host is capable of obtaining the configuration information for wireless communication from the first memory 123, and automatically establishing a communicative connection with the target monitoring device according to the configuration information for wireless communication, without requiring manual operation by the user.

In some embodiments, the mobile monitoring device 12 further includes a contactless communication unit, which is arranged at the monitoring host 100 or the accessory part 200a. The contactless communication unit performs contactless communication to read first configuration information from the target monitoring device in a contactless manner, or transmits first configuration information from the mobile monitoring device 12 to the target monitoring device in a contactless manner. In some embodiments, the contactless communication unit includes at least one of: a near-field communication (NFC) module, a barcode module, a QR code module, a passive/active RFID (radio frequency identification) module, an optical module, an infrared (IR) module, an ultra-wideband (UWB) module, or a magnetic pairing module. In some embodiments, the first configuration information from the mobile monitoring device 12 and the first configuration information from the target monitoring device can be unique device codes of the mobile monitoring device 12 and of the target monitoring device respectively (such as device identification code, device SN code), through which a corresponding relationship list between the mobile monitoring device 12 and the target monitoring device is established to form a pairing. In some embodiments, the first configuration information from the mobile monitoring device 12 and the first configuration information from the target monitoring device can be pairing code(s) randomly generated by the mobile monitoring device 12 and the target monitoring device respectively. The pairing code(s) can be one or more, as long as a corresponding relationship between the mobile monitoring device 12 and the target monitoring device can be established to form a pairing. In some embodiments, the pairing code may be a pairing code from another external third-party device or a manually input pairing code, as long as the mobile monitoring device 12 and the target monitoring device can establish a corresponding relationship therebetween through said pairing code, so as to form a pairing.

In some embodiments, the processor of the mobile monitoring device 12 may be connected with the contactless communication unit and the first memory 123, so as to store, in the first memory 123, first configuration information which is from the target monitoring device and acquired by the contactless communication unit. In some embodiments, the processor of the mobile monitoring device 12 is not connected with the contactless communication unit, and the contactless communication unit is configured to transmit first configuration information of the mobile monitoring device 12 outward, that is, to transmit the first configuration information from the mobile monitoring device 12 to the target monitoring device. After the target monitoring device receives the first configuration information of the mobile monitoring device 12, the target monitoring device establishes a communicative connection with a communication module 111 of the mobile monitoring device 12 based on the first configuration information of the mobile monitoring device 12, and transmits first configuration information of the target monitoring device to the processor of the mobile monitoring device 12 through the communication module 111**,** and then the processor stores in the first memory 123 the first configuration information from the target monitoring device.

**In** some embodiments, the monitoring host 100 further includes a second memory. The second memory is configured to store second configuration information for performing second configuration on the mobile monitoring device 12. The monitoring host 100 is further configured to obtain, from the second memory, at least a portion of the second configuration information and perform second configuration, wherein the second configuration information is at least not completely same as the first configuration information. The second configuration information includes at least one of: configuration information for wireless communication, wearer information, and configuration information for the monitoring host. That is, a portion of information for configuring the monitoring host 100 is stored in the second memory of the monitoring host 100, and a portion of information for configuring the monitoring host 100 is stored in the first memory 123 of the accessory part 200a. The monitoring host 100 obtains configuration information from these two memories for configuration. **In** some embodiments, the first configuration information includes configuration information for wireless communication, and the monitoring host 100 is in communicative connection with the target monitoring device through the first configuration information. The second configuration information includes a configuration parameter for analysis algorithm and a configuration parameter for working mode, and the monitoring host 100 configures its own analysis algorithm and working mode through the second configuration information.

In some embodiments, the monitoring host 100 includes a second memory, which is not configured to store second configuration information for performing second configuration on the mobile monitoring device 12, but is configured to store at least of a portion of first configuration information which is obtained from the first memory 123. That is, the monitoring host 100 obtains at least a portion of the first configuration information from the first memory 123 through the second electrical connection part, then stores the obtained first configuration information in the second memory, and then performs a configuration according to the first configuration information in the second memory.

In an embodiment of this disclosure, the mobile monitoring device 12 also includes a display which is arranged at the monitoring host 100 and configured to display various visual information. The display may be a touch screen, a liquid crystal display, etc.; or, the display may be an independent display, such as a liquid crystal display, a television, etc., which is independent of the mobile monitoring device; or, the display may be a display screen of an electronic device, such as a smart phone or a tablet computer, etc. The number of display(s) may be one or more.

The display can be connected with the processor, and the processor controls the display to display the collected physiological parameter data, and/or a processed result (such as a heart rate value, waveform data, etc.) which is obtained by processing at least a portion of the collected physiological parameter data, in a form of text or graph(s), for the wearer of the mobile monitoring device 12 to view. In an embodiment of this disclosure, the power supply module 112 is configured to provide power to the display.

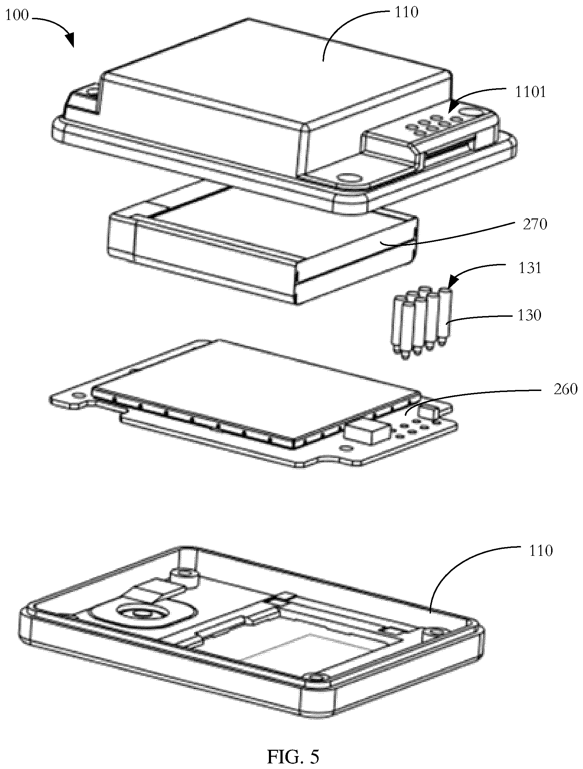

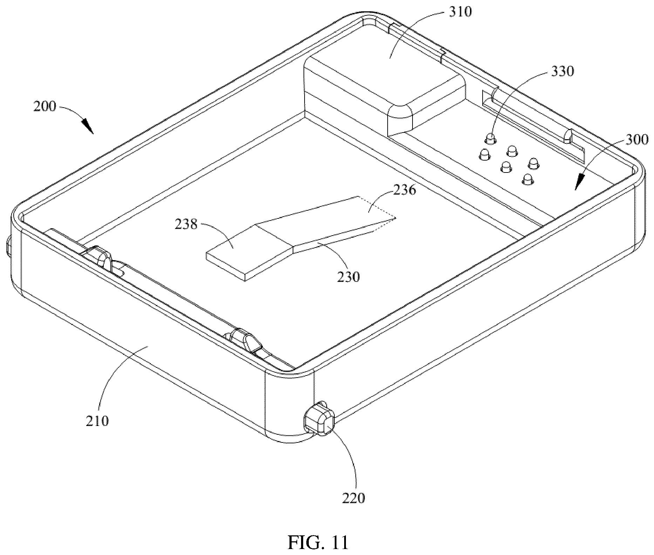

The following describes some exemplary structures of the mobile monitoring device mentioned above with reference to FIGS. 2 to 12, wherein the wearable part mentioned above can be realized via a base 200 mentioned below, and the base 200 can also cooperate with a wearable component 430.

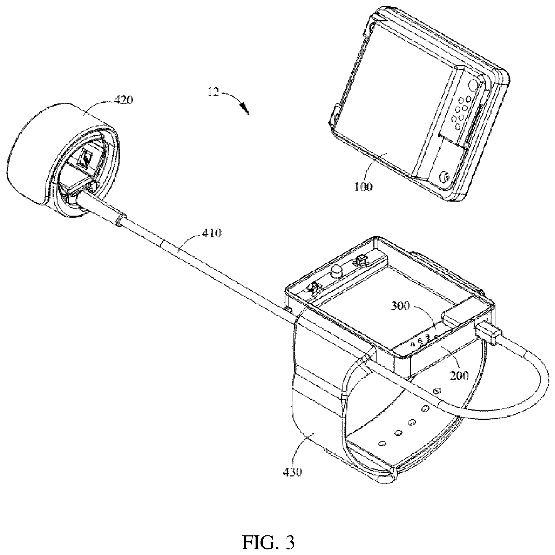

Further, please refer to FIG. 2 and FIG. 3 simultaneously. An embodiment of this disclosure provides a mobile monitoring device 12, which includes a monitoring host 100, a base 200 and a connector 300. The monitoring host 100 is capable of being connected with the base 200, which connection is capable of being disconnected. While realizing a monitoring function, it is also convenient for medical staff to remove the monitoring host 100 when necessary and perform an operation, such as charging, replacing batteries, cleaning and disinfection. The base 200 can cooperate with the wearable component 430 to be fixed to the patient, which fixation is capable of being unfixed. It should be understood that when the monitoring host 100 and the base 200 are connected with each other, which connection is capable of being disconnected, the connector 300 is correspondingly fastened between the monitoring host 100 and the base 200, and is in electrical connection with the monitoring host 100.

In some embodiments, in order to obtain a physiological parameter of a patient, the mobile monitoring device 12 may further include a parameter cable 410 and a sensor 420, wherein the parameter cable 410 is electrically connected between the connector 300 and the sensor 420. The sensor 420 can cooperate with the parameter cable 410, the connector 300 and the monitoring host 100, so as to obtain the physiological parameter of the patient. It should be understood that a connection relationship between the parameter cable 410 and the connector 300 can be a connection which is capable of being disconnected or not capable of being disconnected, and there is no restriction on this.

In some embodiments, the wearable component 430 can be connected with the base 200, so as to allow the user to wear the mobile monitoring device 12. Based on this, in order to achieve wearability, the wearable component 430 can be fixedly connected, or rotatably connected with the base 200, or can be connected with the base 200 which connection is capable of or not capable of being disconnected. Wherein the connection which is not capable of being disconnected may include one-piece molding, adhesive bonding, etc., and there is no restriction on this.

In some embodiments, based on the wearable component 430, the mobile monitoring device 12 can be worn on an arm, wrist, leg, ankle, clothing, or another position of the patient, which position is convenient for measurement. Correspondingly, a type of the wearable component 430 can be a wristband, a clip, a lanyard or a shoulder strap, etc., which is not limited. As shown in FIG. 3, the wearing component 430 includes a pair of wrist straps, which are connected with two sides of the base, so as to be worn on the wrists of the patient.

In some embodiments, the wearable component 430 is further provided with a through opening for a cable (not shown), through which the parameter cable 410 can be passed. Based on this, the parameter cable 410 can be fixed to a certain extent to reduce a possibility of an accident, such as the parameter cable 410 being pulled or deformed by force.

Please refer to FIG. 2, FIG. 4 and FIG. 5. In some embodiments, the monitoring host 100 includes a housing 110, a display screen 101 which is assembled at the housing 110, and a first snap-fit part 120 and a conductive contact 130 which are arranged at the housing 110. The housing 110 may surround and form an inner space of the monitoring host 100. The monitoring host 100 further includes a first circuit board 260 which is arranged inside the internal space. The first circuit board 260 can cooperate with the sensor 420 to monitor a physiological parameter of the patient. In some embodiments, the display screen 101 is a touch display screen, and the processor of the mobile monitoring device 12 switches a parameter value which is displayed on a parameter interface on the display screen 101, in response to a sliding touch operation which is inputted by the user on the display screen 101. In some embodiments, the mobile monitoring device 12 includes a mechanical switch button, and the processor of the mobile monitoring device 12 switches a parameter value which is displayed on a parameter interface on a switching display screen 101, in response to a pressing of the mechanical switch button. In some embodiments, the display screen 101 has a screen locked mode and a screen unlocked mode. When the display screen 101 is in the screen locked mode, a first content interface is displayed; when the display screen 101 is in the screen unlocked mode, a second content interface is displayed; wherein information displayed on the first content interface is different from information displayed on the second content interface. In this way, the display screen 101 of the mobile monitoring device 12 displays different content interfaces respectively in the screen unlocked mode and the screen locked mode, thereby satisfying diverse information viewing requirements of the user. In addition, the first circuit board 260 can also cooperate to realize a function, such as wireless transmission of the physiological parameter.

In order to achieve electrical connection between the first circuit board 260 and related components, the conductive contact 130 has a first end and a second end which are opposite to each other. The first end of the conductive contact 130 is placed inside the internal space of the housing 110 and is electrically connected with the first circuit board 260; the second end of the conductive contact 130 extends toward an external space of the housing 110. It should be understood that the second end of the conductive contact 130 can be in electrical contact with the connector 300 to achieve an electrical connection between the monitoring host 100 and the connector 300. The housing 110 has a surface 1101 on which surface the conductive contact is formed. The conductive contact 130 includes a conductive contact surface 131 which is exposed relative to the housing 110. In this embodiment, the conductive contact surface 131 of the conductive contact 130 is coplanar with the surface 1101 of the housing 110 on which surface the conductive contact 130 is formed, so that an overall outer wall of the housing 110 of the monitoring host 100 is relatively smooth, which further reduces the possibility of dust and liquid accumulation and bacteria breeding on the monitoring host 100, and also reduces a difficulty of cleaning and disinfection. In some embodiments, the surface 1101 of the housing 110, on which surface the conductive contact 130 is formed, is coplanar and higher than the conductive contact surface 131 of the conductive contact 130, that is, the conductive contact 130 is recessed inward relative to the housing 110, which reduces an electrostatic effect of adjacent conductive contacts and acts as a position-limiter to avoid the conductive contact 130 from being offset and causing poor contact. In some other deformable embodiments, the surface 1101 of the housing 110, on which surface the conductive contact 130 is formed, may be coplanar and lower than the conductive contact surface 131 of the conductive contact 130, that is, the conductive contact 130 is arranged to protrude outwardly relative to the housing 110.

In some embodiments, a physiological parameter monitoring module and a power supply module 270 may be integrated into the monitoring host 100 to support a normal operation of the mobile monitoring device 12. Optionally, in some embodiments, the monitoring host 100 also includes, but is not limited to, a storage module and an input/output device. Optionally, the input/output device may be, but not limited to, a wireless transmission module, a USB, etc. The monitoring host 100 can establish a communicative connection with a central station 14, a bedside monitor 16 and a third-party mobile monitoring device 13 through the wireless transmission module, so as to realize wireless transmission of the physiological parameter collected by the sensor 420. Optionally, the physiological parameter collected by the sensor 420 can also be exported from the monitoring host 100 via a USB or a transmission cable. The power supply module 270 may include a power distribution circuit and the above-mentioned battery, wherein the power distribution circuit may convert a voltage provided by a battery into respective working voltage which is required by each functional module.

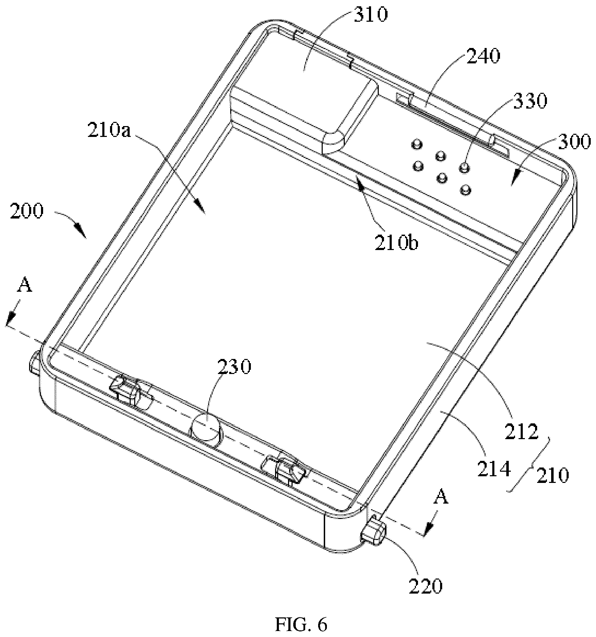

Please refer to FIG. 6. In some embodiments, the base 200 includes a bottom housing 210, and a second snap-fit part 220, and a connector receiving part 210b which are arranged at the bottom housing 210. A position of the second snap-fit part 220 of the base 200 corresponds to a position of the first snap-fit part 120 of the monitoring host 100, thereby achieving a connection between the monitoring host 100 and the base 200, which connection is capable of being disconnected. The connector receiving part 210b can be configured to receive the connector 300. That is, when the monitoring host 100 and the base 200 are connected with each other, which connection is capable of being disconnected, the connector 300 is received in the connector receiving part 210b.

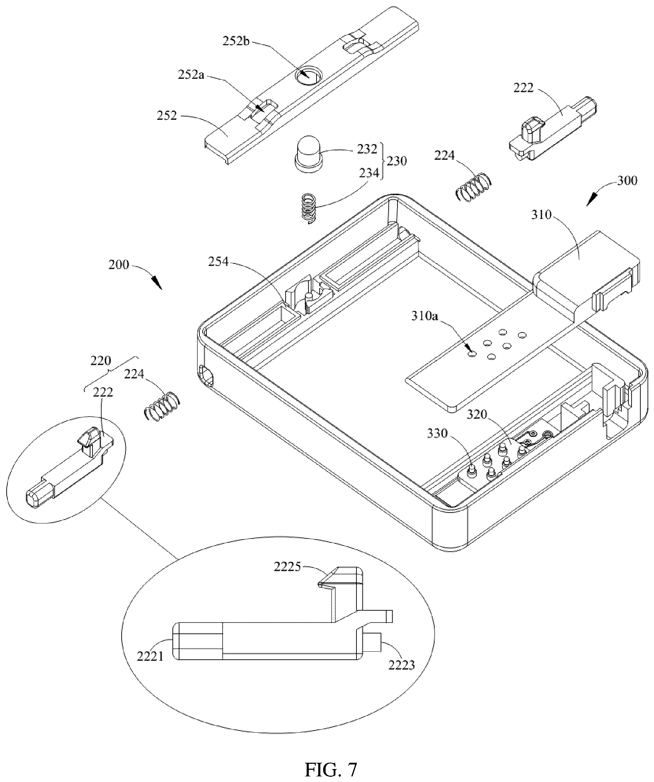

Please refer to FIG. 6 and FIG. 7 simultaneously. In some embodiments, the connector 300 includes a housing 310, and a second circuit board 320 and a conductive pogo pin 330 which are arranged inside the housing 310. The conductive pogo pin 330 may be arranged on the second circuit board 320 and corresponds to the conductive contact 130. The housing 310 has a through hole 310a which corresponds to the conductive pogo pin 330. The conductive elastic pin 330 at least partially passes through the through hole 310a of the housing 310, and a portion of the conductive elastic pin 330, which portion passes through the through hole 310a, is correspondingly located outside the housing 31, so as to be in electrical contact with the conductive contact 130. It should be understood that a number of the conductive pogo pin 330 and a number of the conductive contact 130 may be the same or substantially the same. Under an action of external force, the conductive elastic pin 330 can be retracted relative to the housing 310; and when the external force disappears, the conductive elastic pin 330 can be extended relative to the housing 310.

Based on this, during a connection between the monitoring host 100 and the base 200, which connection is capable of being disconnected, the conductive contact 130 of the monitoring host 100 may contact and apply a force to the conductive elastic pin 330. In contrast, the conductive pogo pin 330 contracts and abuts against the conductive contact 130, thereby achieving an electrical connection between the connector 300 and the monitoring host 100. When the connection between the monitoring host 100 and the base 200, which connection is capable of being disconnected, is disconnected, the conductive pogo pin 330 and the conductive contact 130 is no longer in electrical contact, and the electrical connection between the connector 300 and the monitoring host 100 is simultaneously disconnected. Thus, medical staff can remove the monitoring host 100 to perform an operation, such as charging, replacing batteries, or cleaning and disinfection.

In some embodiments, the external force may include an interaction force which is detected between the host 100 and the base 200 during a connection process between the monitoring host 100 and the base 200, which connection is capable of being disconnected; or, a pressure which is applied to the conductive elastic pin 330 by a technician during a performance test of the conductive elastic pin 330, etc., which is not limited.

In some embodiments, one end of the conductive contact 130 can be electrically connected with the first circuit board 260 by welding, conductive glue, etc.; similarly, the conductive pogo pin 330 can also be electrically connected with the second circuit board 320 by welding, conductive glue, etc., which is not limited.

In some other embodiments, different from the embodiment shown in FIG. 3, positions of the conductive pogo pin and the conductive contact may be swapped, that is, the conductive pogo pin is arranged at the monitoring host 100, and the conductive contact is arranged at the connector 300. It should be understood that this can also achieve an electrical connection between the monitoring host 100 and the connector 300, and facilitate medical staff to perform an operation, such as charging, battery replacement, or cleaning and disinfection, which is not elaborated herein.

It should be understood that, unlike a general mobile monitoring device, in the mobile monitoring device 12 provided in each embodiment of this disclosure, modules for implementing functions are used as relatively independent components according to the different functions implemented. That is, as described above, the mobile monitoring device 12 includes multiple components (100, 200, 300) which are capable of being disassembled, and a surface of each component is relatively smooth to reduce a possibility of dust and liquid accumulation and bacteria breeding. Through the coordination of these multiple components, the normal implementation of the monitoring function of the mobile monitoring device 12 can be ensured, while facilitating the work of medical staff and reducing an intrusion to the patient.

Please synchronously refer to FIG. 6 and FIG. 7 again. In some embodiments, the bottom housing 210 of the base 200 may include a bottom wall 212 and a side wall 214. The side wall 214 is located around the bottom wall 212 and surrounds the bottom wall 212 to form an accommodation cavity 210a. When the monitoring host 100 is connected with the base 200 which connection is capable of being disconnected, the accommodating cavity 210a can correspondingly accommodate at least a portion of the monitoring host 100, and the side wall 214 can protect and assist in fixing the monitoring host 100.

In some embodiments, the connector 300 is fixedly connected with the base 200, and the housing 310 of the connector 300 is a portion of the bottom housing 210 of the base 200. That is, in this embodiment, the connector 300 can be understood as a portion of the base 200. For example, the housing of the connector 300 can be integrally formed with the bottom housing 210 of the base 200, and can also be fixed to the bottom housing 210 of the base 200 by gluing, ultrasonic welding, etc. The conductive contact 130 can be used as a first electrical contact element, the conductive pogo pin 230 can be used as a second electrical contact element, and the first electrical contact element can be in electrical contact with the second electrical contact element, thereby establishing an electrical connection between the monitoring host 100 and the base 200. There are relatively few independent components in the mobile monitoring device 12, so as to facilitate the electrical connection between the monitoring host 100, the connector 300 and the sensor 42.

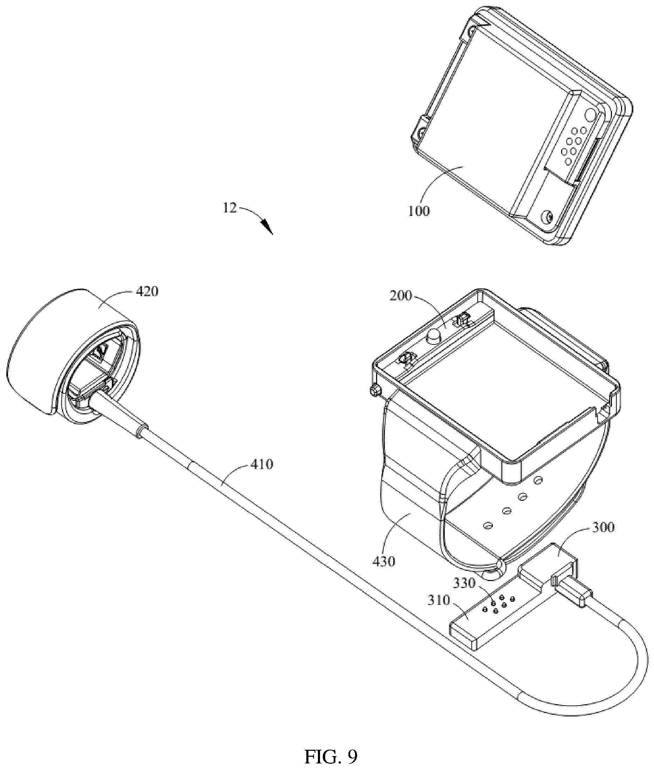

Please refer to FIG. 9. In some other embodiments, the connector 300 is connected with the base 200, which connection is capable of being disconnected. For example, the connector 300 and the base 200 may be connected by means of snap-fit, interference-fit, etc.; or, the connector 300 may be fastened between the monitoring host 100 and the base 200 by means of a fit between the monitoring host 100 and the base 200. It should be understood that in this embodiment, the connector 300 and the base 200 are relatively independent. Based on this, when necessary, medical staff can repair or replace the relatively independent components (100, 200, 300) in the mobile monitoring device 12. For example, the host and the base 200 of the mobile monitoring device 12 are retained, and the connector 300, the parameter cable 410 and the sensor 420 of the mobile monitoring device 12 are replaced, so as to change a type of physiological parameter monitored by the mobile monitoring device 12.

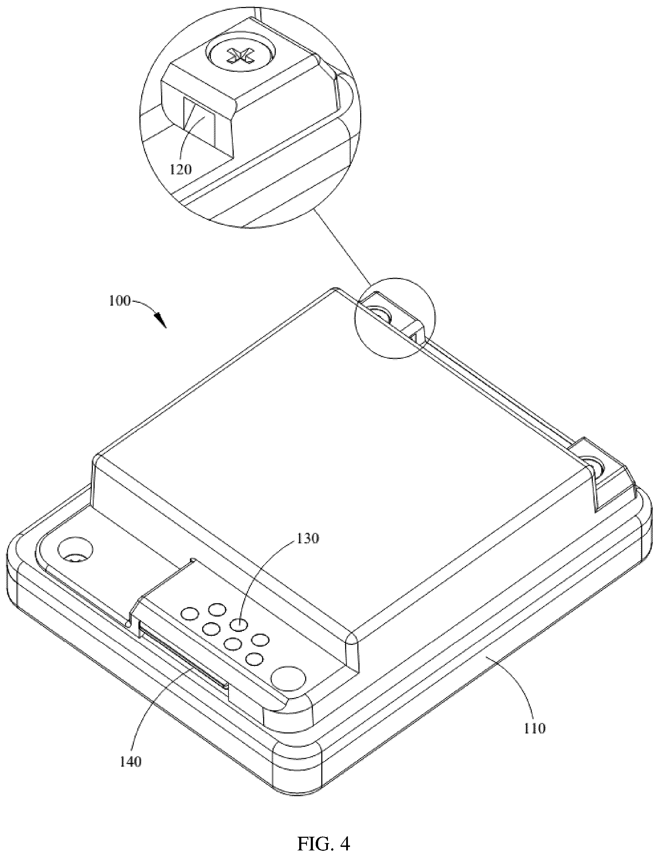

Please refer to FIGS. 4, 6 and 7 again. In some embodiments, since the monitoring host 100 and the base 200 have a connection which is capable of being disconnected, the housing 110 of the monitoring host 100 also includes a first fastener 140, and the bottom housing 210 of the base 200 also includes a second fastener 240 which corresponds to the first fastener 140, so as to improve an effect of a connection between the monitoring host 100 and the base 200, which connection is capable of being disconnected. The first fastener 140 may be a male fastener, and the second fastener 240 may be a female fastener; or, the first fastener 140 may be a female fastener, and the second fastener 240 may be a male fastener.

In some embodiments, the first fastener 140 is located on one side of the housing 110; the first snap-fit part 120 is located on the other side of the housing 110 and is away from the first fastener 140. Corresponding to the first fastener 140 and the first snap-fit part 120, the second fastener 240 is located on one side of the bottom housing 210, the second snap-fit part 220 is located on the other side of the bottom housing 210 and is away from the second fastener 240. Thus, the connection between the monitoring host 100 and the base 200, which connection is capable of being disconnected, can be realized from multiple directions to improve a stability of the connection between monitoring host 100 and the base 200.

In some embodiments, the first snap-fit part 120 may include a position-limiting groove formed by the housing 110. The second snap-fit part 220 may include a button 222 and an elastic element 224, wherein both the button 222 and the elastic element 224 are located at the bottom housing 210. As shown in FIG. 4 and FIG. 7, a number of the first snap-fit part 120 and a number of the second snap-fit part 220 are both two, so as to improve the stability of the connection between the monitoring host 100 and the base 200, however this disclosure is not limited thereto. In some other embodiments, a number of the first snap-fit part 120 and a number of the second snap-fit part 220 may also be one.

Please refer to FIG. 6 and FIG. 7 simultaneously. In some embodiments, the button 222 has a pressing end 2221, an abutting end 2223, and a position-limiting end 2225, which are respectively opposite to each other. The pressing end 2221 is exposed relative to the bottom housing 210, so as to be pressed by medical staff or a patient. Specifically, the pressing end 2221 may extend out of the bottom housing 210; or, may be retracted into the bottom housing 210. Optionally, a pressing surface of the pressing end 2221 is connected and flush with an outer surface of the bottom housing 210, which not only avoids a risk of collision and accidental pressing of the button 222 due to an irregular outer wall, but also makes it convenient for medical staff to clean the base 200. The abutting end 2223 can abut against the elastic element 224 to cooperate and realize a relative movement of the button 222. The position-limiting end 2225 can be connected with the position-limiting groove, which connection is capable of being disconnected, thereby realizing a connection between the base 200 and the monitoring host 100, which connection is capable of being disconnected.

It should be understood that when the relatively independent monitoring host 100 and base 200 need to cooperate to achieve a connection which is capable of being disconnected, medical staff can first insert the first fastener 140 into the second fastener 240 to preliminarily fix a side of the monitoring host 100, which side faces the first fastener 140. Afterwards, with the second fastener 240 of the base 200 as a rotation axis, the medical staff can relatively rotate the monitoring host 100 toward the base 200, until the position-limiting groove is matched with the position-limiting end 2225. Thus, a connection which is capable of being disconnected between the monitoring host 100 and the base 200, can be achieved, and an electrical connection between the conductive contact 130 and the conductive elastic pin 330 can be achieved simultaneously.

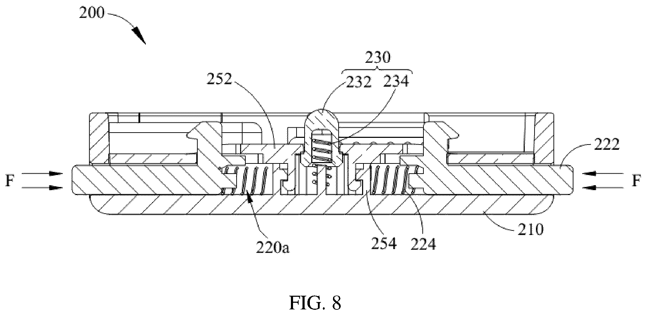

Please refer to FIG. 7 and FIG. 8 simultaneously. When the medical staff presses the pressing end 2221 of the button 222, an external force F applied by the medical staff can make the button 222 slide toward the elastic element 224. The abutting end 2223 can be pressed accordingly to cause the elastic element 224 to generate elastic deformation to store elastic potential energy. The position-limiting end 2225 can be released from the position-limiting groove, so as to enable the monitoring host 100 and the base 200 to be disconnected from the connection which is capable of being disconnected.

When the external force F disappears, the elastically deformed elastic element 224 releases the stored elastic potential energy and causes the button 222 to slide in a direction opposite to the external force, thereby pushing the pressing end 2221 of the button 222 to reset.

Please simultaneously refer to FIGS. 6 and 7. In some embodiments, in order to facilitate medical staff to remove the monitoring host 100 after the connection between the monitoring host 100 and the base 200, which connection is capable of being disconnected, is disconnected, the mobile monitoring device 12 may also include a pop-up mechanism 230 which is arranged at the base 200. After the connection between the monitoring host 100 and the base 200, which connection is capable of being disconnected, is disconnected, the pop-up mechanism 230 can pop out the monitoring host 100 by releasing elastic potential energy or the likes. A number of the pop-up mechanism 230 may be one, two, or more, and there is no restriction on this.

Please simultaneously refer to FIG. 7 and FIG. 8 again. In some embodiments, the pop-up mechanism 230 is arranged at the bottom housing 210. The pop-up mechanism 230 may include a protrusion 232 and an elastic part 234, wherein the elastic part 234 abuts against the protrusion 232 and the bottom housing 210. It should be understood that when the monitoring host 100 is connected with the base 200, which connection is capable of being disconnected, the protrusion 232 can move toward the bottom housing 210 and cause the elastic part 234 to produce elastic deformation due to a force of the monitoring host 100. When the connection between the monitoring host 100 and the base 200, which connection is capable of being disconnected, is disconnected, the elastic part 234 tends to return to a natural state, and correspondingly moves the protrusion 232 away from the bottom housing 210 and separates the monitoring host 100 from the base 200, so that medical staff can take out the monitoring host 100.

In some embodiments, the pop-up mechanism 230 may further include a button cover 252, which is spaced apart from the bottom wall 212 of the bottom housing 210 and may be fixedly connected with the side wall 214 of the bottom housing 210 or with a position-limiting plate 254 to be mentioned below, by ultrasonic welding or the likes. The button 222, the elastic element 224 and the pop-up mechanism 230 may all be arranged between the bottom wall 212 and the button cover 252.

In some embodiments, the button cover 252 is provided with a first opening 252a which can be used for the position-limiting end 2225 of the button 222 to pass through, so as to facilitate a connection with the position-limiting groove of the monitoring host 100. The button cover 252 is further provided with a second opening 252b, which can be used for the protrusion 232 of the pop-up mechanism 230 to pass through, so that the protrusion 232 can abut against the monitoring host 100. Based on the button cover 252, the second snap-fit part 220 and the pop-up mechanism 230 can be integrated together to reduce an overall volume of the base 200 occupied by the second snap-fit part 220and the pop-up mechanism 230, and further facilitating medical staff to clean and disinfect the base 200.

Please simultaneously refer to FIG. 7 and FIG. 8. In some embodiments, the bottom housing 210 may further include a position-limiting plate 254, which is arranged on the bottom wall 212. The position-limiting plate 254 defines a sliding groove 220a. In this embodiment, the position-limiting plate 254 can be integrally formed with the bottom wall 212 or detachably fixedly connected with the bottom wall 212. The position-limiting plate 254, the bottom wall 212 and the side wall 214 may together surround and form a sliding groove 220a. In some other embodiments, the position-limiting plate 254 and the bottom wall 212 may together surround a sliding groove; or, the position-limiting plate 254 and the side wall 214 may together surround a sliding groove; or, the sliding groove 220a is arranged on the position-limiting plate 254 and isolated from the bottom wall 212 and the side wall 214. The button 222 and the elastic element 224 are both arranged inside the sliding groove 220a, and the elastic element 224 abuts against the position-limiting plate 254 and the button 222. It should be understood that a number of the position-limiting plate 254 may vary according to a number of the second snap-fit part 220 and a relative positional relationship between the second snap-fit part 220 and the pop-up mechanism 230, and there is no restriction on this.

As shown in FIG. 8, two second snap-fit parts 220 exist, and the pop-up mechanism 230 is located between the two second snap-fit parts 220. Two position-limiting plates 254 exist, and the two position-limiting plates 254 are fastened between the two second snap-fit parts 220 and the pop-up mechanism 230, so as to ensure a normal realization of their respective functions. In some other embodiments, when the pop-up mechanism 230 is located at another position, the number of the position-limiting plate 254 may be one.

It should be understood that when the monitoring host 100 and the base 200 are connected with each other, which connection is capable of being disconnected, the protrusion 232 can recontract relative to an assembly part through a snap-fit action of the first snap-fit part 120 and the second snap-fit part 220, so that the elastic part 234 generates elastic deformation. When the connection between the monitoring host 100 and the base 200, which connection is capable of being disconnected, is disconnected, the elastic part 234 tends to return to a natural state due to an absence of a restriction of the first snap-fit part 120 and the second snap-fit part 220, and an elastic force acts on the protrusion 232 and causes the protrusion 232 to pop out of the monitoring host 100. In some embodiments, the elastic part 234 may include a compression spring, elastic foam, a gas bag, etc., which is not limited.

It should be understood that when the monitoring host 100 and the base 200 are connected with each other, which connection is capable of being disconnected, the protrusion 232 recontracts toward an assembly cavity and causes the elastic part 234 to produce elastic deformation. When the connection between the monitoring host 100 and the base 200, which connection is capable of being disconnected, is disconnected, the elastic part 234 is used to enable the protrusion 232 to extend out of the assembly cavity and separate the monitoring host 100 from the base 200.

In some embodiments, an extension and retraction direction of the conductive pogo pin 330 is same as a movement direction of the pop-up mechanism 230, thereby cooperating to pop up the monitoring host 100. It should be understood that the extension and retraction direction and the movement direction generally refer to a direction perpendicular to the surface of the bottom wall 212.

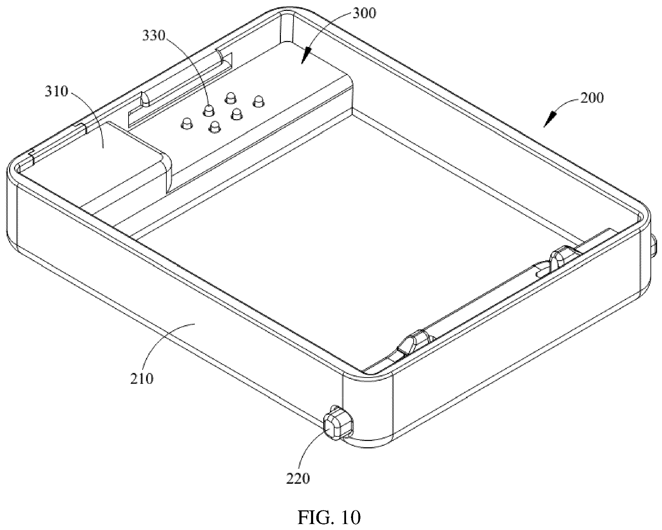

Please refer to FIG. 10. In some other embodiments, the mobile monitoring device 12 may not include the pop-up mechanism 230. In order to pop the monitoring host 100 up, the conductive elastic pin 330 may have a certain elastic force. After the connection between the monitoring host 100 and the base 200, which connection is capable of being disconnected, is disconnected, the conductive elastic pin 330 can pop up the monitoring host 100 from the base 200 to achieve separation, thereby making it easier for medical staff to remove the monitoring host 100. In addition, the conductive pogo pin 330 can also improve the reliability of the electrical connection with the conductive contact 130 of the monitoring host 100.

In some embodiments, the pop-up mechanism 230 may include an element for storing elastic energy or a gas bag. The element for storing elastic energy may include a spring sheet.

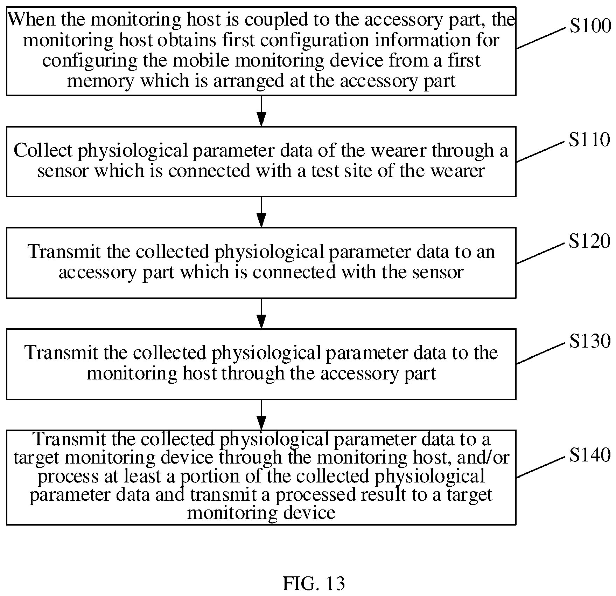

Please refer to FIG. 13, in some embodiments, the pop-up mechanism 230 is a spring sheet. The pop-up mechanism 230 has a fixed end 236 and an elastic end 238 which are opposite to each other. The fixed end 236 is fixed to the bottom housing 210, and the elastic end 238 extends in a direction away from the bottom housing 210 and protrudes relative to the bottom housing 210. When the monitoring host 100 is connected with the base 200, which connection is capable of being disconnected, the elastic end 238 moves toward the bottom housing 210, and the spring sheet generates an elastic deformation. When the connection between the monitoring host 100 and the base 200, which connection is capable of being disconnected, is disconnected, the elastic end 238 can move in a direction away from the bottom housing 210 to separate the monitoring host 100 and the base 200.

In some embodiments, the housing 110 may further include a groove (not shown) corresponding to the protrusion 232. When the monitoring host 100 is connected with the base 200, which connection is capable of being disconnected, the groove can accommodate the protrusion 232. Based on this, after the monitoring host 100 is popped up, a relative position of the monitoring host 100 and the base 200 can be limited by a cooperation between the protrusion 232 and the groove and the cooperation between the first fastener 140 and the second fastener 240, so as to reduce a possibility of the monitoring host 100 for falling off accidentally.

In some other embodiments, the pop-up mechanism includes a protrusion, an elastic part, and an assembly part; the assembly part is assembled at the bottom housing and surrounds the bottom housing to form an assembly cavity. The elastic part and the protrusion are sequentially assembled inside the assembly cavity, and the elastic part abuts against the protrusion and the bottom housing. Based on this, the pop-up mechanism can be located at any position of the bottom wall of the bottom housing, and does not need to be arranged close to the second snap-fit part.

In some other embodiments, the second snap-fit part 220 may include a position-limiting groove. The first snap-fit part 120 may include a button 222, an elastic element 224 and so on, which are all arranged at the housing 110. Based on this, same technical effects as those in the above embodiments can also be achieved, which is not elaborated herein.

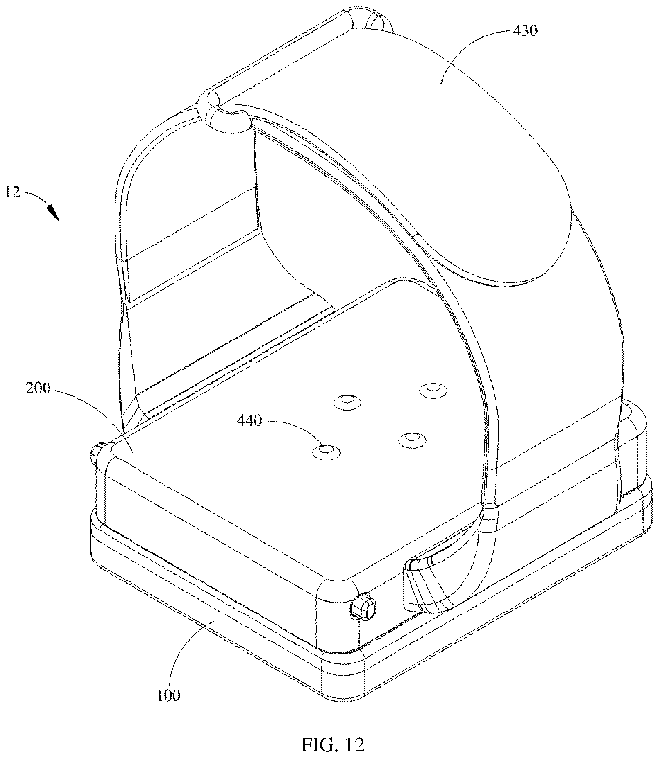

Please refer to FIG. 12. In some embodiments, different from the parameter cable 410 and the sensor 420 described above, the mobile monitoring device 12 may further include an optical transceiver assembly 440. The optical transceiver assembly 440 is arrange on a side of the base 200, which side is away from the accommodating cavity 210a. The optical transceiver assembly 440 may be electrically connected with the connector 300 and configured to obtain a physiological parameter of a user after the user wears the mobile monitoring device 12. It should be understood that, in this embodiment, a conductive path may be arranged at the base 200 to ensure a relatively stable electrical connection between the connector 300 and the optical transceiver assembly 440.

In some embodiments, the optical transceiver component 440 may include a light emitting element and a light receiving element. The light emitting element can irradiate lights of different wavelengths onto skin of the patient. The lights are reflected by human tissue and received by the light receiving element, thereby measuring a physiological parameter, such as blood oxygen, a pulse rate, a blood pressure, and a body temperature.

With reference to FIG. 13, a method for collecting and transmitting physiological parameter data of a wearer according to an embodiment of this disclosure is exemplarily described.

The method is applied to a mobile monitoring device, which includes a monitoring host, an accessory part and a sensor. The monitoring host includes a power supply module and a communication module. The communication module can be a wireless communication module, which may include at least one of a Bluetooth module, a WMTS (Wireless Medical Telemetry Services) communication module, a contactless (full-range) communication module and a WIFI communication module. The power supply module may include a rechargeable battery, which is used to supply power to components which are inside the mobile monitoring device and require power. The monitoring host is coupled to the accessory part and communicates with the accessory part 200a, which coupling is capable of being decoupled. Wherein the accessory part includes a wearable part and a first memory, wherein the wearable part is fixed to a designated body part of the wearer which fixation is capable of being unfixed, the first memory stores first configuration information for performing first configuration on the mobile monitoring device. The sensor is connected with the accessory part for collecting physiological parameter data of the wearer. The sensor can be one or more of: a blood oxygen probe (which includes a light emitting element and a light receiving element), an ECG electrode, a body temperature sensor, and a blood pressure sensor. Accordingly, the physiological parameter data collected by the sensor includes one or more of: a blood oxygen parameter, an ECG parameter, a body temperature parameter, and a blood pressure parameter. **In** some embodiments, the sensor may be communicatively coupled to the accessory part via a data cable. Exemplarily, the mobile monitoring device may also be implemented as the mobile monitoring device described above.

**In** some embodiments, the method for collecting and transmitting physiological parameter data of a wearer includes following steps.

S100, when the monitoring host is coupled to the accessory part, the monitoring host obtains first configuration information for configuring the mobile monitoring device from a first memory which is arranged at the accessory part.

**In** an embodiment of this disclosure, the first configuration information may include at least one of: configuration information for wireless communication, wearer information, and configuration information for the monitoring host. Wherein, the configuration information for wireless communication may include at least one of: a frequency, a communication channel, a baud rate, an apparatus identification number, an MAC address, an **IP** address and a communication port, wherein the apparatus identification number can be a randomly generated pairing code or a unique and fixed apparatus code for each apparatus. The wearer information includes at least one of: a gender, a classification, an age, a height, a weight, a bed number, a department, and a medical history. The configuration information for the monitoring host may include at least one of: a sensor type, a configuration parameter for analysis algorithm, and a configuration parameter for working mode. **In** some embodiments, in step S100, after the monitoring host obtains the first configuration information, the monitoring host performs first configuration according to the first configuration information. Wherein, performing the first configuration may include: establishing, by the monitoring host, a wireless communicative connection with a target monitoring device through a communication module, according to the configuration information for wireless communication which information is used for pairing with the target pairing device. Performing the first configuration may also include: configuring analysis algorithm and working mode of the monitoring host, by the monitoring host, according to the configuration parameter for analysis algorithm and the configuration parameter for working mode. After the monitoring host performs the first configuration according to the first configuration information, subsequent steps are performed

S110, collect physiological parameter data of the wearer through a sensor which is connected with a test site of the wearer.

S120, transmit the collected physiological parameter data to an accessory part which is connected with the sensor.

S130, transmit the collected physiological parameter data to the monitoring host through the accessory part.

S140, transmit the collected physiological parameter data to a target monitoring device through the monitoring host, and/or process at least a portion of the collected physiological parameter data and transmit a processed result to a target monitoring device.

In an embodiment of this disclosure, the mobile monitoring device also includes a processor. The processor is arranged inside the monitoring host or the accessory part, and the processor is in communicative connection with the sensor and the communication module. In step S140, the processor controls the communication module to transmit the collected physiological parameter data to a target monitoring device, and/or processes at least a portion of the collected physiological parameter data, and controls the communication module to transmit a processed result to a target monitoring device. In some embodiments, in step S140, the processor may process ECG parameter data collected by the sensor to obtain a heart rate value, waveform data, etc., and then control the communication module to transmit the obtained heart rate value and waveform data to the target monitoring device. In some embodiments, in step S140, the processor can determine whether arrhythmia occurs based on a preset rule and the acquired ECG parameter data. When arrhythmia is determined to occur, an arrhythmia alarm event is generated, and then the communication module is controlled to transmit the arrhythmia alarm event to the target monitoring device so that medical staff can handle it in time. In step S140, for other physiological parameter data except the ECG parameter data, the processor can process such data in a similar manner and control the communication module to transmit the processed result to the target monitoring device, which is not described in detail here. The processor may be a single chip microcomputer or other electronic device with computing and processing capabilities.

In an embodiment of this disclosure, the method for collecting and transmitting physiological parameter data of a wearer further includes a following step.

S150, when the monitoring host is coupled to the accessory part, supply power to the sensor through a power supply module which is arranged at the monitoring host.

In an embodiment of this disclosure, the method for collecting and transmitting physiological parameter data of a wearer further includes a following step.

S160, when the monitoring host is coupled to the accessory part, supply power to the first memory by a power supply module which is arranged at the monitoring host.

In some embodiments, the monitoring host further includes a second memory. The second memory is configured to store second configuration information for performing second configuration on the mobile monitoring device. The second configuration information may include at least one of: configuration information for wireless communication, wearer information, and configuration information for the monitoring host.

Accordingly, the method for collecting and transmitting physiological parameter data of a wearer further includes a following step.

S170, obtain second configuration information which is stored in a second memory which is arranged at the monitoring host, and perform second configuration on the monitoring host based on the second configuration information; wherein the second configuration information is at least not completely same as the first configuration information.

In an embodiment of this disclosure, the method for collecting and transmitting physiological parameter data of a wearer further includes following steps.

S180, while a mobile monitoring device establishes a communicative connection with a target monitoring device for the first time, perform a pairing operation between the mobile monitoring device and the target monitoring device.

It should be noted that "a first time" here refers to a first time for a new patient; "mobile monitoring device" in step 180, refers to a mobile monitoring device 12 which is worn by the new patient for the first time, that is, a first monitoring host, which is coupled to an accessory part which is worn on a new patient for the first time, as well as said accessory part, together form said mobile monitoring device. In some embodiments, the processor controls the communication module to perform a pairing operation between the mobile monitoring device and the target monitoring device. In some embodiments, a process for the processor to perform the pairing operation between the mobile monitoring device and the target monitoring device requires manual operation by medical staff, for example, the medical staff needs to manually input a pairing code on the mobile monitoring device or the target monitoring device.

S181, after the mobile monitoring device and the target monitoring device have successfully paired with each other, establish a communicative connection between the mobile monitoring device and the target monitoring device, obtain the first configuration information, and output the first configuration information to the first memory for storing.

The first configuration at least includes a pairing operation between a mobile monitoring device and a target monitoring device, and the first configuration information at least includes configuration information for wireless communication, which information is required to perform the pairing operation between the mobile monitoring device and the target monitoring device. After the first configuration information is stored in the first memory, when the monitoring host is subsequently replaced, the newly replaced monitoring host can obtain, from the first memory, the configuration information for wireless communication, and automatically establish a communicative connection with the target monitoring device according to the configuration information for wireless communication without requiring manual operation by the user.

In some embodiments, the mobile monitoring device further includes a contactless communication unit, which is arranged at the monitoring host or the accessory part, and is used for contactless communication with the target monitoring device. Accordingly, the method for collecting and transmitting physiological parameter data of a wearer further includes a following step.

S190, perform contactless communication through a contactless communication unit which is arranged at the monitoring host, so as to read the first configuration information from the target monitoring device in a contactless manner, or to transmit the first configuration information from the mobile monitoring device to the target monitoring device, in a contactless manner.

Wherein, the contactless communication part can be an NFC (Near Field Communication) module, and the target monitoring device is also correspondingly provided with an NFC module. The NFC module can be used to transmit or receive the first configuration information. Thus, through the contactless communication between the NFC modules respectively at the mobile monitoring device and the target monitoring device, the first configuration information from the target monitoring device can be read by the mobile monitoring device, or the first configuration information from the mobile monitoring device can be transmitted to the target monitoring device. In some embodiments, a processor of the mobile monitoring device may be connected with the contactless communication unit and the first memory to store, in the first memory, the first configuration information, which is from the target monitoring device and acquired by the contactless communication unit. In some embodiments, the processor of the mobile monitoring device is not connected with the contactless communication unit, and the contactless communication unit is configured to transmit first configuration information of the mobile monitoring device outward, that is, to transmit said first configuration information from the mobile monitoring device to the target monitoring device. After the target monitoring device receives said first configuration information of the mobile monitoring device, the target monitoring device establishes a communicative connection with the communication module of the mobile monitoring device based on said first configuration information of the mobile monitoring device 12, and transmits first configuration information of the target monitoring device to the processor of the mobile monitoring device through the communication module, and the processor then stores in the first memory said first configuration information from the target monitoring device.

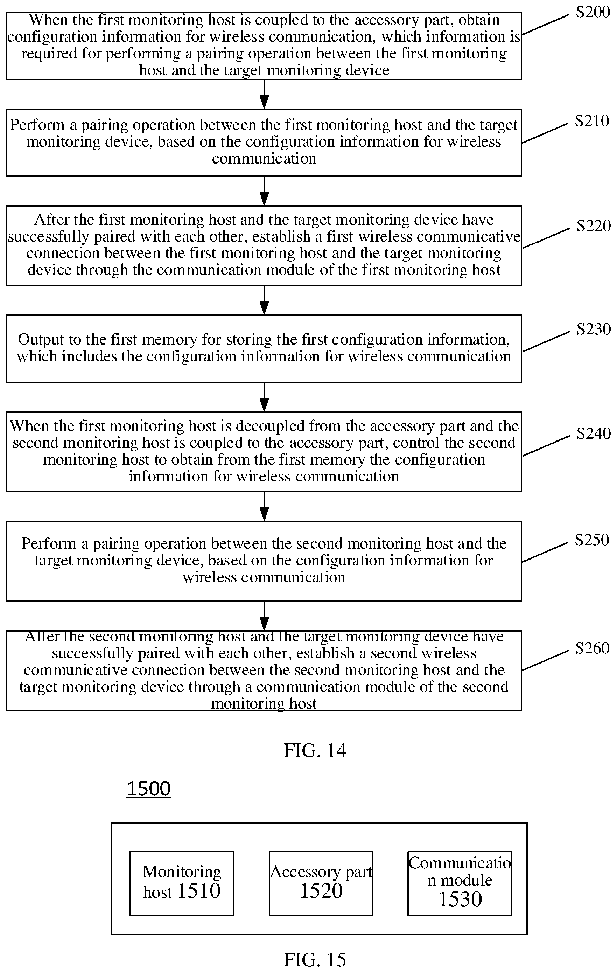

In some embodiments, the mobile monitoring device includes at least a first monitoring host and a second monitoring host, both of which include a battery unit and a communication module, and are capable of being coupled to the accessory part and communicate with the accessory part, which coupling is capable of being decoupled. When power of the first monitoring host, which is coupled to the accessory part, is low, the first monitoring host can be decoupled from the accessory part, and a second monitoring host can be coupled to the accessory part to continue the monitoring process. The first monitoring host is a monitoring host that is coupled to the accessory part for the first time. The accessory part includes a wearable part and a first memory. The mobile monitoring device is fixed to a designated body part of the wearer through a wearable part, which fixation is capable of being unfixed. The mobile monitoring device also includes a sensor, which is in communicative connection with the accessory part. Accordingly, as shown in FIG. 14, the method for collecting and transmitting physiological parameter data of a wearer may include following steps S200 to S260.

S200, when the first monitoring host is coupled to the accessory part, obtain configuration information for wireless communication, which information is required for performing a pairing operation between the first monitoring host and the target monitoring device.

In an embodiment of this disclosure, a contactless communication unit is arranged at the first monitoring host. The contactless communication unit may include at least one of: a near-field communication (NFC) module, a barcode module, a QR code module, a passive/active RFID (radio frequency identification) module, an optical module, an infrared (IR) module, an ultra-wideband (UWB) module, or a magnetic pairing module. In step S200, configuration information for wireless communication is obtained by the first monitoring host from the target monitoring device in a contactless manner through the contactless communication unit; or, configuration information for wireless communication is obtained from the first monitoring host, and then transmitted to the target monitoring device in a contactless manner through the contactless communication unit. Wherein, the configuration information for wireless communication includes at least one of: a device identification code, a device SN (serial number) code, a randomly generated pairing code, a frequency, a communication channel, a baud rate, an apparatus identification number, an MAC address, an IP address, a service set identifier (referred to as SSID), a WEP (Wired Equivalent Privacy) protocol, a WPA (Wi-Fi Protected Access) protocol, a WPA2 protocol, a WPA3 protocol, an EAP (Extensible Authentication Protocol) protocol, other protocol-related information, and a communication port. In an embodiment of this disclosure, when the first monitoring host is coupled to the accessory part, the sensor and the first memory in the accessory part are powered by a power supply module arranged at the first monitoring host.

S210: perform a pairing operation between the first monitoring host and the target monitoring device, based on the configuration information for wireless communication.

Specifically, in some embodiments, after the first monitoring host obtains the configuration information for wireless communication from the target monitoring device in a contactless manner, the configuration information for wireless communication is broadcasted through the communication module of the first monitoring host, so as to enable the communication module of the target monitoring device to establish a first wireless communicative connection with the first monitoring host, when the configuration information for wireless communication is detected by the wireless communication module of the target monitoring device. In some embodiments, after the first monitoring host transmits configuration information for wireless communication to the target monitoring device in a contactless manner through the contactless communication unit, the communication module of the first monitoring host detects the configuration information for wireless communication, and establishes a first wireless communicative connection with the target monitoring device, when the configuration information for wireless communication, which is broadcasted by the communication module of the target monitoring device, is detected.

S220, after the first monitoring host and the target monitoring device have successfully paired with each other, establish a first wireless communicative connection between the first monitoring host and the target monitoring device through the communication module of the first monitoring host.

Wherein, the communication module of the first monitoring host can be at least one of a Bluetooth module, a WMTS communication module, a contactless communication module and a WIFI communication module. The target monitoring device is provided with a communication module corresponding to the communication module of the first monitoring host. After the communication module of the first monitoring host establishes a first wireless communicative connection with the target monitoring device, the first monitoring host can transmit the physiological parameter data collected by the sensor at the accessory part to the target monitoring device through the communication module, and/or process at least a portion of the collected physiological parameter data, and transmit a processed result to the target monitoring device through the communication module.

S230, output to the first memory for storing the first configuration information, which includes the configuration information for wireless communication.

In an embodiment of this disclosure, the accessory part is provided with a first electrical connection part, and the first monitoring host is provided with a second electrical connection part. The first monitoring host and the accessory part communicate with each other through the first electrical connection part and the second electrical connection part. Thus, the first monitoring host can output first configuration information which includes configuration information for wireless communication to the first memory for storing, through the first electrical connection part and the second electrical connection part. The wireless configuration information which is outputted to the first memory may be configuration information for wireless communication of a communication module which is arranged at the target monitoring device. In addition to the configuration information for wireless communication, the first configuration information also includes at least one of: wearer information, and configuration information for the monitoring host. The wearer information includes at least one of: a gender, a classification, an age, a height, a weight, a bed number, a department, and a medical history; the configuration information for the monitoring host includes at least one of: a sensor type, a configuration parameter for analysis algorithm and a configuration parameter for working mode.

S240, when the first monitoring host is decoupled from the accessory part and the second monitoring host is coupled to the accessory part, control the second monitoring host to obtain from the first memory the configuration information for wireless communication.

In an embodiment of this disclosure, the accessory part is provided with a first electrical connection part, and the first monitoring host is provided with a second electrical connection part. The first monitoring host and the accessory part communicate with each other through the first electrical connection part and the second electrical connection part. Thus, when the first monitoring host is decoupled from the accessory part and the second monitoring host is coupled to the accessory part, a processor which is arranged at the second monitoring host or at the accessory part can control the second monitoring host to obtain from the first memory the configuration information for wireless communication (that is, the configuration information for wireless communication of the target monitoring device) stored in the first memory through the first electrical connection part and the second electrical connection part.

S250, perform a pairing operation between the second monitoring host and the target monitoring device, based on the configuration information for wireless communication.

In an embodiment of this disclosure, a processor, which is arranged at the second monitoring host or at the accessory part, controls a communication module of the second monitoring host to perform a pairing operation with the target monitoring device based on the configuration information for wireless communication, that is the configuration information for wireless communication of the target monitoring device.

S260, after the second monitoring host and the target monitoring device have successfully paired with each other, establish a second wireless communicative connection between the second monitoring host and the target monitoring device through a communication module of the second monitoring host.

Wherein, the communication module of the second monitoring host can be at least one of: a Bluetooth module, a WMTS communication module, a contactless communication module and a WIFI communication module. The target monitoring device is provided with a communication module which corresponds to the communication module of the second monitoring host. In some embodiments, the configuration information for wireless communication is broadcasted by the communication module of the second monitoring host, so as to enable the communication module of the target monitoring device to establish a second wireless communicative connection with the second monitoring host, when the configuration information for wireless communication is detected by the communication module of the target monitoring device. In some embodiments, the communication module of the second monitoring host detects the configuration information for wireless communication, and establishes a second wireless communicative connection with the target monitoring device, when the configuration information for wireless communication, which is broadcasted by the communication module of the target monitoring device, is detected. After the communication module of the second monitoring host establishes a second wireless communicative connection with the target monitoring device, the second monitoring host can transmit the physiological parameter data collected by the sensor at the accessory part to the target monitoring device through the communication module, and/or process at least a portion of the collected physiological parameter data and transmit a processed result to the target monitoring device through the communication module.

Referring to FIG. 15, a mobile monitoring device 1500 according to an embodiment of this disclosure is exemplarily described. The mobile monitoring device 1500 is fixed to a wearer, which fixation is capable of being unfixed, the mobile monitoring device 1500 is configured to collect physiological parameter data of the wearer. The mobile monitoring device 1500 is in communicative connection with a target monitoring device, so as to transmit the collected physiological parameter data to the target monitoring device. The target monitoring device may be one or more of: a bedside monitor, a central station, a department-level workstation device, and a hospital-level data center/a management device of a hospital-level emergency center. The mobile monitoring device 1500 includes a monitoring host 1510, an accessory part 1520, and a communication module 1530. For some details of the mobile monitoring device 1500, reference may be made to the description of the mobile monitoring device above.

The monitoring host 1510 includes a power supply module, which is assembled at the monitoring host 1510, which assembly is capable of being dis assembled. In some embodiments, the power supply module may include a rechargeable battery, which can be used to power components in the mobile monitoring device 1500 that require power. The monitoring host 1510 and the accessory part 1520 are connected with each other, which connection is capable of being disconnected. The accessory part 1520 at least includes a wearable part that is in close contact with a body part of the wearer, a sensor, and a first memory.

The wearable part can be implemented as the aforementioned base, which can be used in conjunction with wearable components such as wristbands and clips, and is used to be fixed to a designated part of a body of the user (such as a wrist, arm, etc.), which fixation is capable of being unfixed. Thus, when the monitoring host 1510 is coupled to the accessory part 1520, the mobile monitoring device 1500 can be fixed to a designated body part of the wearer through the wearable part, which fixation is capable of being unfixed. Since the monitoring host 1510 and the accessory part 1520 are coupled, which coupling is capable of being decoupled; when the monitoring host 1510 is low on power, medical staff can remove the monitoring host 1510 separately for replacement without removing the wearable part from the body of the user, thereby reducing intrusion to the user.

The sensor is configured to be connected with a test site of the wearer to collect corresponding physiological parameter data. In some embodiments, the sensor can be one or more of: a blood oxygen probe (which includes a light emitting element and a light receiving element), an ECG electrode, a body temperature sensor, and a blood pressure sensor. Accordingly, the physiological parameter data collected by the sensor includes one or more of: a blood oxygen parameter, an ECG parameter, a body temperature parameter, and a blood pressure parameter. In some embodiments, the physiological parameter data collected by the sensor may be one or more of: a respiratory parameter, a sleep parameter, a motion parameter, and a pain parameter. In some embodiments, the parameter cable is a cable used for data transmission, one end of which is connected with the sensor and the other end of which is connected with the wearable part, so as to transmit the physiological parameter data collected by the sensor to the wearable part, and then to the monitoring host 1510. In some embodiments, the parameter cable is connected with the sensor, which connection is not capable of being disconnected, for example, the parameter cable is fixedly connected with the sensor, by welding, gluing, etc. In some embodiments, the parameter cable is connected with the sensor, which connection is capable of being disconnected, for example, the parameter cable is connected with the sensor via a plug connector and a plug slot, which connection is capable of being disconnected. In some embodiments, the parameter cable is connected with the wearable part, which connection is not capable of being disconnected, for example, the parameter cable is fixedly connected with the wearable part by welding, gluing, etc. In some embodiments, the parameter cable is connected with the wearable part, which connection is capable of being disconnected, for example, the parameter cable is connected with the wearable part via a plug connector and a plug slot, which connection is capable of being disconnected.

The first memory is configured to store first configuration information for performing first configuration on the monitoring host 1510. Wherein, the first configuration information may include at least one of: configuration information for wireless communication, wearer information, and configuration information for the monitoring host. Wherein, the configuration information for wireless communication may include at least one of: a device identification code, a device SN (serial number) code, a randomly generated pairing code, a frequency, a communication channel, a baud rate, an MAC address, an **IP** address, a service set identifier (referred to as SSID), a WEP (Wired Equivalent Privacy) protocol, a WPA (Wi-Fi Protected Access) protocol, a WPA2 protocol, a WPA3 protocol, an EAP (Extensible Authentication Protocol), and other protocol-related information, and a communication port; wherein the apparatus identification number can be a randomly generated pairing code or a unique and fixed apparatus code for each apparatus. The wearer information includes at least one of: a gender, a classification, an age, a height, a weight, a bed number, a department, and a medical history. The configuration information of the monitoring host 1510 may include at least one of: a sensor type, a configuration parameter for analysis algorithm, and a configuration parameter for working mode. In some embodiments, after the newly replaced monitoring host 1510 obtains the first configuration information and performs corresponding configuration, it can continue the work performed by a monitoring host 1510 before replacement, thereby ensuring the continuity of the monitoring process and the consistency of the monitoring result.

The communication module 1530 is configured to establish a communicative connection between the mobile monitoring device 1500 and the target monitoring device. The communication module 1530 is arranged at the monitoring host 1510 or the accessory part 1520. The communication module 1530 may be a wireless communication module, which may include at least one of: a Bluetooth module, a WMTS (Wireless Medical Telemetry Services) communication module, a contactless (full-range) communication module, and a WIFI communication module.

In an embodiment of this disclosure, the power supply module is configured to supply power to the sensor and the first memory, when the monitoring host 1510 is coupled to the accessory part 1520.

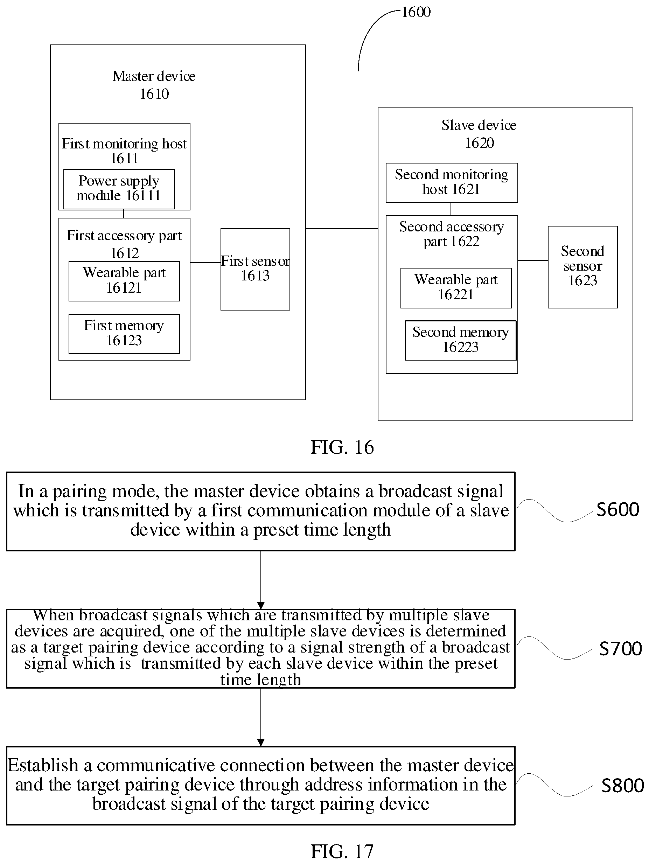

In another embodiment of this disclosure, a mobile monitoring system 1600 is also provided. As shown in FIG. 16, the mobile monitoring system 1600 also includes a master device 1610 and at least one slave device 1620, wherein at least one of the master device 1610 and the slave device 1620 can be implemented via the mobile monitoring device described above, and some details about each device can be referred to the above description.

As an example, as shown in FIG. 16, the master device 1610 includes: a first monitoring host 1611 and a first accessory part 1612; the first accessory part 1612 may include a wearable part 16121, the master device 1610 is fixed to a designated body part of the wearer through the wearable part 16121, which fixation is capable of being unfixed; the first monitoring host 1611 and the first accessory part 1612 are in communicative connection with each other. The first monitoring host 1611 includes a communication module and a power supply module, wherein the master device 1610 and a target monitoring device communicate with each other through a communication module, for example, the first monitoring host 1611 is coupled to the first accessory part 1612 and communicates with the first accessory part 1612, which coupling is capable of being decoupled.

Exemplarily, the master device 1610 also includes a first sensor 1613 and a parameter cable, wherein the first sensor 1613 is connected with a test site of the wearer to collect corresponding physiological parameter data; wherein one end of the parameter cable is connected with the sensor, and the other end of the parameter cable is connected with the first accessory part 1612 to transmit the physiological parameter data collected by the first sensor 1613 to the accessory part.

The master device 1610 may include a first memory 16123, which may be arranged at any position of the first accessory part 1612, such as a wearable part (such as a base, a strap, etc.), a parameter cable, or a sensor. Optionally, the first memory 16123 may be arranged at any position of the first monitoring host 1611.

In one example, the master device 1610 includes a power supply module 16111, which is assembled inside the first monitoring host 1611 or inside the first accessory part 1612, which assembly is capable of being disassembled. The power supply module may include a rechargeable battery, etc., to supply power to components of the master device 1610 that require power. Further, in some examples, when the power supply module is arranged inside the first monitoring host 1611, the power supply module is capable of being in electrical connection with the first memory 16123 so as to supply power to the first memory 16123, when the first monitoring host 1611 and the first accessory part 1612 are coupled.

At least one slave device 1620 is in communicative connection with the master device 1610, wherein a wireless communicative connection or a wired communicative connection may be established between the master device 1610 and the slave device 1620. The master device 1610 and the slave device 1620 may share a host for monitoring; or, may respectively use different hosts for monitoring.

The master device 1610 is in wireless connection with at least one of a bedside monitor and a central station. The master device 1610 can be used as a HUB (multi-port repeater) in a human body local area network, and can transmit data such as a physiological parameter outward. It should be understood that both the master device 1610 and the slave device 1620 can obtain corresponding physiological parameters from the patient and transmit these physiological parameters to the master device 1610 in a timely manner. According to these physiological parameters, the master device 1610 can transmit these physiological parameters to the central station or the bedside monitor, so as to enable the central station or the bedside monitor to analyze a physical condition of the patient based on these physiological parameters, and provide an analysis result to medical staff for them to take corresponding measures; or the master device 1610 can implement all or portion of analysis of the physiological parameters locally, and transmit an analysis result to the central station or the bedside monitor for medical staff to take corresponding measures.

Exemplarily, each slave device 1620 includes a second monitoring host 1621, and a second accessory part 1622, wherein the second monitoring host 1621 and the second accessory part 1622 are in communicative connection with each other. For example, the second monitoring host 1621 is coupled to the second accessory part 1622 and communicates with the second accessory part 1622, which coupling is capable of being decoupled.

The second accessory part 1622 may include a wearable part 16222, through which the slave device 1620 is fixed to a designated body part of the wearer, which fixation is capable of being unfixed. The second monitoring host 1621 and the second accessory part 1622 are in communicative connection with each other, the second monitoring host 1621 includes a communication module and a power supply module, wherein the slave device and the target monitoring device communicate with each other through the communication module, for example, the second monitoring host 1621 is coupled to the second accessory part 1622 and communicates with the second accessory part 1622, which coupling is capable of being decoupled.

Exemplarily, the slave device 1620 also includes a second sensor 1623, which is connected with a test site of the wearer to collect corresponding physiological parameter data. The second sensor 1623 can be arranged at the second monitoring host 1621 or the second accessory part 1622, or can also be connected with the second accessory part 1622 via a cable.

At least one slave device 1620 further includes a second memory 16223. At least one of the second accessory part 1622 and the second monitoring host 1621 is provided with the second memory 16223 which can be used to store any information that needs to be stored in the slave device 1620.

The first memory 16123 and the second memory 16223 may be non-volatile memories. For example, the non-volatile memories may include a read-only memory (ROM), a hard disk, a flash memory, and the likes.

In some examples, the first memory 16123 is configured to store first configuration information of the first monitoring host 1611 and/or configuration information for slave device of the second monitoring host 1621, and the second memory 16223 is configured to store the first configuration information of the first monitoring host 1611 and/or configuration information for slave device of the second monitoring host 1621, wherein, after a first monitoring host 1611 is replaced, a new first monitoring host 1611 for replacing is capable of obtaining the first configuration information from the first memory 16123 for configuration, or obtaining the first configuration information from the second memory 16223 of one or more of the slave devices 1620 for configuration, or, obtaining a portion of the first configuration information from the first memory 16123 and obtaining another portion of the first configuration information from the second memory 16223 for configuration.

In some embodiments, after replacing a second monitoring host 1621 of a slave device 1620, a new salve device 1620 for replacing is capable of obtaining the configuration information for slave device from the first memory 16123 for configuration, or obtaining the configuration information for slave device from the second memory 16223 of another slave device 1620 for configuration, or obtaining a portion of the configuration information for slave device from the first memory 16123 and another portion of the configuration information for slave device from the second memory 16223 of another slave device 1620 for configuration.

By using the above method, when replacing the monitoring host of the master device 1610 or of the slave device 1620, there is no need to perform wireless pairing and another operation, which is more convenient and quicker.

In some embodiments, the first configuration information includes at least one of: configuration information for wireless communication, wearer information, and configuration information for the monitoring host; configuration information for slave device includes at least one of: configuration information for wireless communication, wearer information, and configuration information for the monitoring host. Wherein, the wearer information includes at least one of: a gender, a classification, an age, a height, a weight, a bed number, a department, and a medical history or other information related to the wearer, and the configuration information for the monitoring host includes at least one of: a sensor type, a configuration parameter for analysis algorithm, a configuration parameter for working mode; the configuration information for wireless communication includes at least one of: a device identification code, a device SN (serial number) code, a randomly generated pairing code, a frequency, a communication channel, a baud rate, an MAC address, an IP address, a service set identifier (referred to as SSID), a WEP (Wired Equivalent Privacy) protocol, a WPA (Wi-Fi Protected Access) protocol, a WPA2 protocol, a WPA3 protocol, an EAP (Extensible Authentication Protocol), and other protocol-related information, and a communication port.

In some embodiments, the first monitoring host 1611 of the master device 1610 also includes a third memory, wherein the third memory is configured to store second configuration information, and the first monitoring host 1611 is capable of obtaining the second configuration information from the third memory for configuration. The second configuration information may not be exactly same as the first configuration information. For example, the second configuration information includes at least one of: configuration information for wireless communication, wearer information, and configuration information for the monitoring host. In particular, the second configuration information may include general information, such as configuration information for department.

In some embodiments, the master device 1610 and the slave device 1620 are configured to obtain physiological parameter data, wherein the physiological parameter data include at least one of: an electrocardiogram parameter value, a respiratory parameter value, a blood oxygen parameter values, a blood pressure parameter value, a body temperature parameter value, a sleep parameter value, a motion parameter value, a pain parameter value, or other parameter data that needs to be monitored.

In some embodiments, the master device 1610 also includes a first processor, which is arranged inside the first accessory part 1612 or the first monitoring host 1611, and the first processor is also configured to control to transmit to the target monitoring device real-time data and historical data of the physiological parameter data collected by the mobile monitoring system 1600, when the mobile monitoring system 1600 and the target monitoring device are in communicative connection with each other, so that the target monitoring device can present the physiological parameter data to the user.

In some embodiments, the first processor is also configured to perform a pairing operation between the master device 1610 and the target monitoring device, while the master device 1610 establishes a communicative connection with the target monitoring device for the first time; and establish a communicative connection between the master device 1610 and the target monitoring device, obtain first configuration information, and output the first configuration information to the first memory 16123 and/or the second memory 16223 for storing, after the master device 1610 and the target monitoring device have successfully paired with each other.

It is worth mentioning that the target monitoring device may also be a slave device 1620 that is to perform wireless communication with the master device 1610, or may also be a bedside monitor or a central station.

In some embodiments, the first processor is further configured to generate information to indicate pairing, so as to indicate the user whether to pair the mobile monitoring device with the target monitoring device, before executing the pairing operation with the target monitoring device; and configured to execute the pairing operation with the target monitoring device when obtaining a selection instruction.

Optionally, when a first monitoring host 1611 is replaced with another first monitoring host 1611, the processor which is arranged inside the first accessory part 1612 or said another first monitoring host 1611 does not generate information to indicate pairing, that is, a display of the master device 1610 does not display information to indicate pairing, and the processor directly performs the pairing operation.

In one embodiment, the slave device 1620 further includes a second processor, which is arranged inside the second accessory part 1622 or the second monitoring host 1621.

In one embodiment, the mobile monitoring system 1600 further includes a display, which is configured to display various visual information, such as operation interface information, data information, etc.

Further, referring to FIG. 17, a method for wireless pairing of a mobile monitoring device according to an embodiment of this disclosure is exemplarily described.

The mobile monitoring device in an embodiment of this disclosure includes a master device and a slave device. At least one of the master device and the slave device can be fixed to a body of a user (e.g., a patient), which fixation is capable of being unfixed, so as to obtain basic physiological parameters of the user (e.g., electrocardiogram, body temperature, etc.) for achieving mobile monitoring of the user. The master device is provided with a processor and a first communication module, and the slave device is provided with a first communication module. Wherein, the first communication module can be one of: a Bluetooth communication module, a Wi-Fi communication module, and a WMTS (wireless medical telemetry services) communication module. The processor may be a single chip microcomputer or another device or component with computing processing capability.

The method for wireless pairing of a mobile monitoring device includes following steps.

In S600, in a pairing mode, the master device obtains a broadcast signal which is transmitted by a first communication module of a slave device within a preset time length.

Specifically, the first communication module of the slave device may be configured to continuously transmit a broadcast signal at a certain time interval. The broadcast signal includes address information, which may be used for an external device to pair with the slave device to establish a communicative connection. When the master device is in a pairing mode, the processor acquires, through the first communication module, a broadcast signal which is transmitted by the first communication module of the slave device and received within a preset time length (e.g., 500 milliseconds, 1 second, or other suitable time). In some embodiments, when the master device is triggered to enter into a pairing mode, the processor obtains, through the first communication module, a broadcast signal which is transmitted by the first communication module of the slave device within a preset time length (e.g., 500 milliseconds, 1 second, or other suitable time) after entering into a pairing mode. It should be noted that the time interval for transmitting the broadcast signal from the first communication module of the slave device is less than the preset time length. In some embodiments, when the master device is not in a pairing mode, the master device does not receive the broadcast signal which is transmitted by the slave device (or other external device) and cannot establish a communicative connection with the slave device; when the master device enters into a pairing mode, the master device starts to receive a broadcast signal which is transmitted by the slave device (or other external device).

In S700: when broadcast signals which are transmitted by multiple slave devices are acquired, one of the multiple slave devices is determined as a target pairing device according to a signal strength of a broadcast signal which is transmitted by each slave device within the preset time length.

Specifically, since the first communication module of the slave device is configured to continuously transmit broadcast signals at a certain time interval, the master device may obtain broadcast signals which are transmitted by multiple slave devices in a pairing mode, among which slave devices, usually only one slave device is a target pairing device that the master device needs to pair with. The master device determines the target pairing device from the multiple slave devices according to a signal strength of a broadcast signal which is transmitted by each slave device within a preset time length, that is, according to a signal strength of a broadcast signal which is received by the master device within a preset time length.

In some embodiments, step S700 includes following steps.

In S210, calculate a statistical value of the signal strength of the broadcast signal which is transmitted by each slave device within a preset time length. Specifically, the processor in the master device performs a statistical calculation on a signal strength of a broadcast signal which is transmitted by each slave device and received through the first communication module within a preset time length, so as to obtain a statistical value, which is used to characterize a quality of a signal strength for each broadcast signal. In some embodiments, the statistical value may be an average value of the signal strength of the broadcast signal within the preset time length. In some embodiments, the statistical value may be a median value of the signal strength of the broadcast signal within the preset time length. In some embodiments, the statistical value may be a peak value of a histogram of the signal strength of the broadcast signal within the preset time length.

In S220, compare statistical values of signal strengths of the multiple broadcast signals, and determine the target pairing device according to a comparison result.

Specifically, the processor in the master device compares the statistical values of the signal strengths of the multiple broadcast signals, for example, ranks the statistical values in order from large to small or from small to large, so as to determine a broadcast signal with a best signal strength (greatest statistical value), and then determines as a target pairing device a slave device which transmits said broadcast signal.

In some embodiments, step S220 includes following steps:
S221, rank the statistical values in order from large to small or from small to large;
S222, obtain a difference between a maximum statistical value among the statistical values and a statistical value which is ranked adjacent to the maximum statistical value; that is, to obtain a difference between a greatest statistical value and a second greatest statistical value;
S223, determine whether a current pairing is successful based on a relationship of size between the difference and a preset threshold range. Specifically, in step S223, when the difference is greater than or equal to the preset threshold range, the current pairing is determined to be successful, and the slave device which corresponds to the maximum statistical value is used as the target pairing device. When the difference is less than the preset threshold range, the current pairing is determined to have failed. It should be noted that the success of pairing here refers to the processor successfully determining the target pairing device (the slave device corresponding to the maximum statistical value), rather than successfully establishing a communicative connection. In some embodiments, the preset threshold range may refer to a preset single threshold.

Since the signal strength of the broadcast signal which is transmitted by the slave device is not necessarily stable, the signal strength of the target pairing device may not be optimal. That is to say, when the difference between the greatest statistical value and the second greatest statistical value is very small, it is impossible to accurately determine the slave device corresponding to which statistical value is the target pairing device. If the slave device corresponding to the greatest statistical value is directly paired as the target pairing device, there is a possibility of mispairing. Through the above steps S221-S223, the target pairing device can be accurately determined, effectively reducing the possibility of mispairing.

In some embodiments, when the difference in step S223 is less than a preset threshold range and it is determined that the current pairing fails, step S600, step S210 and steps S221-S223 are performed again. It should be noted that re-performing step S600 means that in the pairing mode, the master device obtains a broadcast signal which is transmitted by the first communication module of the slave device within a next preset time length.

S800, establish a communicative connection between the master device and the target pairing device through address information in the broadcast signal of the target pairing device.

Specifically, since the broadcast signal, which is transmitted from a first communication module of the slave device, includes address information, after determining the target pairing device, the processor can establish a communicative connection with the target pairing device according to the address information in the broadcast signal which is transmitted from the target pairing device. For a Bluetooth communication module, a Wi-Fi communication module or a WMTS communication module, when the address information is known, the specific process of establishing a communicative connection is known to those skilled in the art and is not described in detail here. In some embodiments, when a communicative connection is established between the master device and the target pairing device, the target pairing device can transmit information (such as a measurement result of a sensor at the target pairing device) to the master device through a first communication module. In some embodiments, when a communicative connection is established between the master device and the target pairing device, the target pairing device can transmit information (such as a measurement result of a sensor at the target pairing device) to the master device through a first communication module, and the master device can also transmit information (such as a control signal for a sensor at the target pairing device) to the target pairing device through a first communication module.

Generally, the closer the distance between the master device and the slave device, the stronger the signal strength of the broadcast signal which is transmitted by the slave device and obtained by the master device. Therefore, for the method for wireless pairing of the mobile monitoring device in an embodiment of this disclosure, it is only necessary to ensure that the slave device (target pairing device) to be paired with the master device is within a very close distance to the master device, when the master device is in pairing mode; then, a communicative connection between the master device and the target pairing device can be automatically established. There is no need for the master device (or slave device) to display a list of devices which have been searched for, nor for the user to select a corresponding device in a displayed device list, which effectively reduces operation process of the user, effectively reduces the probability of mispairing, and improves the usability of the product. In some embodiments, even if the master device (or slave device) does not have a display interface or have a display interface without interactive capabilities, the pairing process can be conveniently implemented through the above pairing method.

In some embodiments, the master device is capable of detecting whether communication of the second communication module exists, according to a preset period, wherein a communication distance of the second communication module is less than 100 cm. A second communication module is arranged at the slave device that needs to be paired with the master device (i.e., the target pairing device), and is used to trigger the master device to enter into a pairing mode. The master device detecting that communication of the second communication module exists, indicates that the master device and the target pairing device are already in a very close distance. At this time, the master device can accurately establish a wireless communicative connection with the target pairing device through the above steps S600-S800. That is to say, the user only needs to place the master device and the target pairing device in a very close distance (less than 100 cm), and the master device can automatically establish a communicative connection with the target pairing device. The operation is very simple and convenient.

Accordingly, before step S600, the method for performing wireless pairing of the mobile monitoring device further includes a following step.

In S10, the master device enters into a pairing mode, when detecting communication of the slave device, which device is provided with the second communication module. Wherein, the slave device, which is provided with the second communication module, is a target pairing device. After the master device enters into the pairing mode, the above steps S600-S800 are performed.

In some embodiments, the second communication module may be an NFC (Near Field Communication) tag, which is attached to the target pairing device. Correspondingly, the master device is also provided with an NFC module, which is configured to detect whether communication of an NFC tag exists according to a preset period. In some embodiments, the NFC module at the master device may be configured to operate in an active mode, so as to transmit a radio frequency signal according to a preset period and detect whether a response is received from an NFC tag. The NFC tag attached to the target pairing device can be configured to work in a passive mode, and only transmit a corresponding response when receiving the radio frequency signal which is transmitted by the NFC module at the master device. When the NFC module of the master device receives the response, it enters into the pairing mode. In some embodiments, the NFC tag may also be built into the target pairing device.

In some embodiments, a physical button is provided at the master device, and configured to trigger the master device to enter into the pairing mode (when pressed). After the master device enters into the pairing mode, the above steps S600-S800 are performed. That is to say, the user only needs to bring the master device and the target pairing device very close to each other and press the physical button on the master device, and the master device can automatically establish a communicative connection with the target pairing device. The operation is very simple and convenient.

Accordingly, before step S600, the method for performing wireless pairing of the mobile monitoring device further includes a following step.

In S20, trigger the master device to enter into the pairing mode in response to an operation on the physical button.

Specifically, in some embodiments, the physical button is configured to control connection and disconnection of a detection circuit (for example, being disconnected when the physical button is not pressed, and being connected when the physical button is pressed), and the processor determines whether an operation on the physical button (whether the physical button is pressed) exists based on the connection and disconnection of the detection circuit. When an operation on the physical button is determined (the physical button is pressed), in response to the operation on the physical button, the master device is controlled to enter into the pairing mode. After the master device enters into the pairing mode, the above steps S600-S800 are performed.

In some embodiments, the master device is provided with an operation interface, which may be a display interface of a touch screen. The operation interface is provided with a pairing mode option, and the user may control the master device to enter into the pairing mode by selecting the option. After the master device enters into the pairing mode, the above steps S600-S800 are performed. That is to say, the user only needs to bring the master device and the target pairing device very close to each other and select the pairing mode option on the operation interface of the master device, and the master device can automatically establish a communicative connection with the target pairing device. The operation is very simple and convenient.

Accordingly, before step S600, the method for performing wireless pairing of the mobile monitoring device further includes a following step.

In S30, trigger the master device to enter into the pairing mode, in response to a selection operation on an pairing mode option.

Specifically, in some embodiments, the touch screen of the master device displays an operation interface, and the touch screen is also configured to detect a user operation on the touch screen. When the processor at the master device determines that the pairing mode option on the display interface is selected based on a detection result of the touch screen, the processor controls the master device to enter into the pairing mode in response to the selection operation of the pairing mode option. After the master device enters into the pairing mode, the above steps S600-S800 are performed.

In some embodiments, the master device also includes a microphone, which is configured to receive a voice control signal. The processor of the master device is configured to determine whether the voice control signal is a preset voice control signal. When the voice control signal is a preset voice control signal, the master device is controlled to enter into the pairing mode. After the master device enters into the pairing mode, the above steps S600-S800 are performed. That is to say, the user only needs to bring the master device and the target pairing device very close to each other and speak a specific voice (preset voice control signal), and the master device can automatically establish a communicative connection with the target pairing device. The operation is very simple and convenient.

Accordingly, before step S600, the method for performing wireless pairing of the mobile monitoring device further includes a following step.

In S40, the master device enters into the pairing mode, when obtaining a voice control signal for controlling entry into the pairing mode.

Specifically, in some embodiments, the processor of the master device determines whether the voice control signal received through the microphone is a preset voice control signal for controlling the master device to enter into the pairing mode, and if yes, controls the master device to enter into the pairing mode. After the master device enters into the pairing mode, the above steps S600-S800 are performed.

In some embodiments, when the master device is not in the pairing mode, the processor of the master device receives broadcast signals which are transmitted from the slave device through the first communication module, and obtains a signal strength of each broadcast signal, and determines whether a signal strength of at least one broadcast signal exceeds a preset threshold signal strength. If yes, the master device is controlled to enter into the pairing mode. After the master device enters into the pairing mode, the above steps S600-S800 are performed. That is to say, the user only needs to place the master device and the target pairing device very close to each other, and the master device can automatically establish a communicative connection with the target pairing device. The operation is very simple and convenient.

Accordingly, before step S600, the method for performing wireless pairing of the mobile monitoring device further includes a following step.

In S50, trigger the master device to enter into the pairing mode, when determining that a signal strength of an acquired broadcast signal, which signal is transmitted by the slave device, is greater than a threshold signal strength.

According to the method for wireless pairing of a mobile monitoring device of this disclosure, the master device determines the target pairing device based on the signal strength of the broadcast signal which is transmitted by the slave device within a preset time length, and establishes a communicative connection with the target pairing device through the address information in the broadcast signal of the target pairing device. During the pairing process, user operations can be effectively reduced, the pairing process is simplified, and the probability of incorrect pairing is reduced.

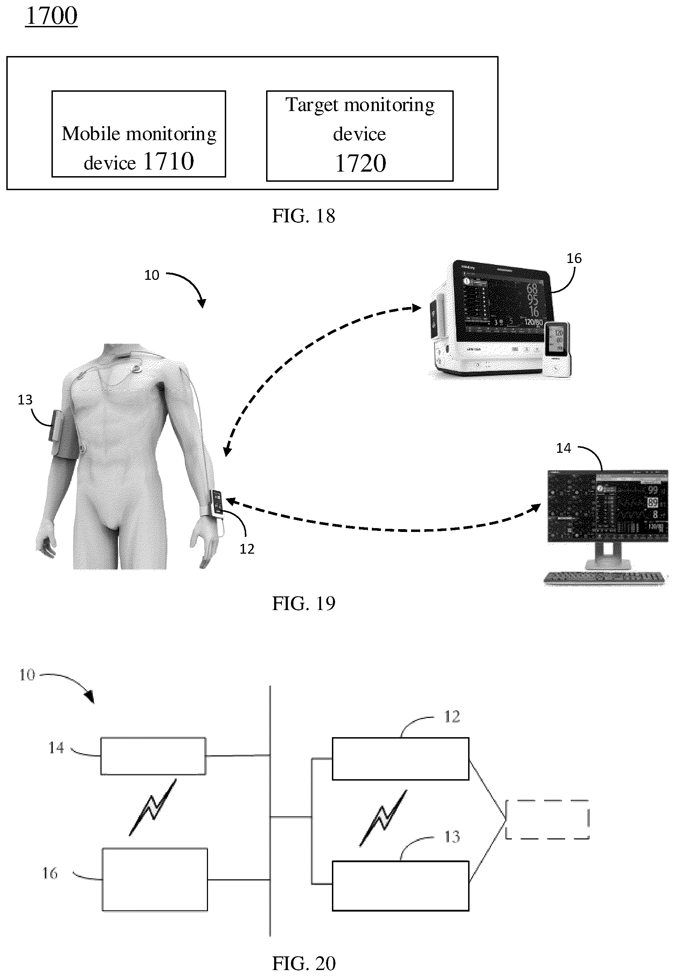

Furthermore, this disclosure also provides a monitoring system, which is described below with reference to FIGS. 18 to 21.

As shown in FIG. 18, the monitoring system 1700 may include a mobile monitoring device 1710 and a target monitoring device 1720.

The mobile monitoring device 1710 may be the mobile monitoring device 100 or the mobile monitoring device 1500 in the above-mentioned embodiment.

The mobile monitoring device 1710 is fixed to a wearer, which fixation is capable of being unfixed, and is configured to collect physiological parameter data of the wearer. Specifically, the mobile monitoring device 1710 is provided with a sensor, which is connected with a test site of the wearer so as to collect corresponding physiological parameter data. In some embodiments, the sensor can be one or more of a blood oxygen probe (which includes a light emitting element and a light receiving element), an ECG electrode, a body temperature sensor, and a blood pressure sensor. Accordingly, the physiological parameter data collected by the sensor includes one or more of: a blood oxygen parameter, an ECG parameter, a body temperature parameter, and a blood pressure parameter. In some embodiments, the physiological parameter data collected by the sensor may be one or more of: a respiratory parameter, a sleep parameter, a motion parameter, and a pain parameter.

The mobile monitoring device 1710 is also configured to transmit real-time data and historical data of the physiological parameter data collected by the mobile monitoring device 1710 (i.e., the sensor) to the target monitoring device, when the mobile monitoring device 1710 and the target monitoring device 1720 are in communicative connection. In some embodiments, the mobile monitoring device 1710 is further configured to process at least a portion of the acquired physiological parameter data and transmit a processed result to the target monitoring device 1720.

The target monitoring device 1720 may be one or more of: a bedside monitor, a central station, a department-level workstation device, and a hospital-level data center/a management device of a hospital-level emergency center. The target monitoring device 1720 and the mobile monitoring device 1710 are in communicative connection. The target monitoring device 1720 and the mobile monitoring device 1710 can achieve communicative connection through at least one of: a Bluetooth module, a WMTS (Wireless Medical Telemetry Services) communication module, a contactless communication module and a WIFI communication module. The specific manner in which the target monitoring device 1720 and the mobile monitoring device 1710 establish a communicative connection can refer to the above embodiment and is not repeated here. The target monitoring device 1720 is provided with a display screen, and is configured to obtain the physiological parameter data collected by the mobile monitoring device 1710, and display the physiological parameter data through the display screen, so as to enable the target monitoring device 1720, such as a central station or a bedside monitor, to analyze a physical condition of the patient according to these physiological parameter data, and provide an analysis result to medical staff for them to take corresponding measures. Alternatively, the mobile monitoring device 12 implements all or a portion of the analysis of the physiological parameter data locally, and transmits an analysis result to the central station or the bedside monitor for the medical staff to take corresponding measures.

In one embodiment, as shown in FIG. 19, the monitoring system may also be implemented as a monitoring system 10, which includes a mobile monitoring device 12. Each mobile monitoring device 12 can be fixed to a patient, which fixation is capable of being unfixed, and obtain a corresponding type of physiological parameter. The mobile monitoring device 12 may include multiple detachable parts, such as a wearable part, a data processing part, etc. A battery may be arranged at the data processing part. Based on this, when the mobile monitoring device 12 needs to be charged or the battery needs to be replaced, the data processing part (such as the monitoring host) can be removed and related operations can be performed, while the wearable part can still be worn on the patient, so as to reduce the intrusion to the patient and reduce the difficulty of the work of medical staff.

The mobile monitoring device 12 is configured to obtain from the user a physiological parameter which corresponds to a sensor type. The target monitoring device includes at least one of: a bedside monitor 16 and a central station 14. The bedside monitor 16 is in wireless connection with the mobile monitoring device 12. The bedside monitor 16 is configured to receive the physiological parameter which is transmitted by the mobile monitoring device 12. The central station 14 is in respective wireless connection with the bedside monitor 16 and with the mobile monitoring device 12. The central station 14 communicates with the bedside monitor 16 to transmit physiological parameter; or, receives the physiological parameter transmitted by the mobile monitoring device 12.

It can be understood that, the monitoring system 10 of this disclosure is applicable to a single-patient usage scenario and a multi-patient usage scenario. Specifically, please refer to FIG. 19 and FIG. 20, FIG. 20 is a diagram of a single-patient usage scenario for the mobile medical monitoring system 10. The monitoring system 10 includes a mobile monitoring device 12, a third-party mobile monitoring device 13, a central station 14, and a bedside monitor 16. A structure of the mobile monitoring device 12 is different from that of the third-party mobile monitoring device 13. The third-party mobile monitoring device 13 is a mobile monitoring device existing in the medical industry. A wireless communicative connection or a wired communicative connection may be established between the mobile monitoring device 12 and the third-party mobile monitoring device 13. The mobile monitoring device 12 and the third-party mobile monitoring device 13 may share a host for monitoring; may respectively use different hosts for monitoring.

Any one of the mobile monitoring device 12 and the third-party mobile monitoring device 13 serves as a master device, and the other serves as a slave device. The master device is in a wireless connection with at least one of the bedside monitor 16 and the central station 14. The master device can be used as a HUB (multi-port repeater) in the human body local area network, and can transmit data outward, such as physiological parameters. It should be understood that both the mobile monitoring device 12 and the third-party mobile monitoring device 13 can obtain corresponding physiological parameters from the patient and transmit these physiological parameters to the master device in a timely manner. Based on these physiological parameters, the master device can transmit these physiological parameters to the central station or bedside monitor, so as to enable the central station or bedside monitor to analyze a physical condition of a patient based on these physiological parameters and provide the analysis result to medical staff for them to take corresponding measures; or, the master device can implement portion or all of analysis of the physiological parameters locally, and transmit the analysis result to the central station or bedside monitor for medical staff to take corresponding measures.

For example, in a single-patient usage scenario, a user may wear one mobile monitoring device 12 and one third-party mobile monitoring device 13 at the same time. When the mobile monitoring device 12 serves as the master device, the master device is configured to receive a physiological parameter which is collected by the third-party mobile monitoring device 13, and to transmit physiological parameters which are respectively collected by the mobile monitoring device 12 and the third-party mobile monitoring device 13 to at least one of the bedside monitor 16 and the central station 14. When the third-party mobile monitoring device 13 serves as the master device, the master device is configured to receive a physiological parameter which is collected by the mobile monitoring device 12, and to transmit physiological parameters which are respectively collected by the mobile monitoring device 12 and the third-party mobile monitoring device 13 to at least one of the bedside monitor 16 and the central station 14. It can be understood that when the monitoring system 10 is applied to a single-patient usage scenario, the monitoring system 10 may include multiple mobile monitoring devices 12 and multiple third-party mobile monitoring devices 13. Optionally, multiple mobile monitoring devices 12 may be in wireless communicative connection with each other. The multiple third-party mobile monitoring devices 13 may be in wireless communicative connection with one or more of the mobile monitoring devices 12.

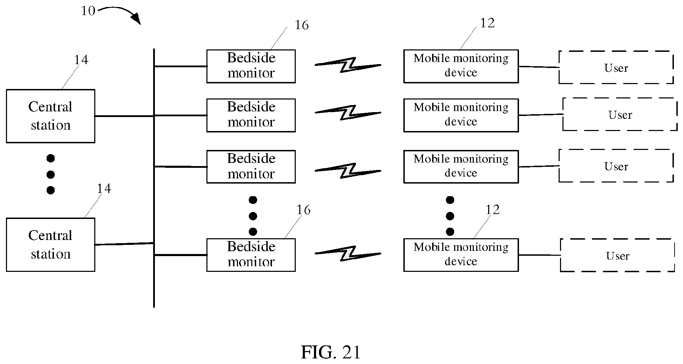

Please refer to FIG. 21, which is a diagram of a multi-patient usage scenario for the mobile medical monitoring system 10. The monitoring system 10 includes multiple mobile monitoring devices 12, at least one central station 14, and at least one bedside monitor 16. The multiple mobile monitoring devices 12 may establish communicative connections with at least one central station 14 and at least one bedside monitor 16, respectively. For example, in a multi-patient usage scenario, one user wears one mobile monitoring device 12, and physiological parameters which are collected by each mobile monitoring device 12 can be transmitted to the corresponding bedside monitor 16 and the corresponding central station 14, so that medical staff can take corresponding measures.

In addition, in the monitoring system 10, multiple mobile monitoring devices 12 can also independently transmit data, such as a physiological parameter, to a central station or a bedside monitor without transmitting them together through a master device. It should be understood that, with respect to physiological parameter, the above two transmission modes can be flexibly converted and implemented without limitation.

In some embodiments, a type of a physiological parameter which is monitored by the mobile monitoring device 12 may include: a blood oxygen parameter, an electrocardiogram parameter, a blood pressure parameter, a respiration parameter, a body temperature parameter, etc., which is not limited.

As shown in FIG. 19, a number of mobile monitoring devices 12 is two, and the two mobile monitoring devices 12 are in wirelessly communicative connection with each other. The mobile monitoring device 12 on the right is a blood oxygen monitoring device, and the mobile monitoring device 12 on the left is an electrocardiogram monitoring device. It should be understood that the blood oxygen monitoring device can be worn on the wrist of the patient to monitor the blood oxygen parameter of the patient. The ECG monitoring device can be fixed to clothes of the patient by means of a clip or the like, or can be directly attached to skin of the patient to monitor the ECG parameter of the patient.

In some embodiments, the blood oxygen monitoring device is used as a master device, and the blood oxygen monitoring device can transmit a collected blood oxygen parameter as well as, an electrocardiogram parameter which is collected by the electrocardiogram monitoring device, to the central station 14 or the bedside monitor 16. In some other embodiments, the blood oxygen monitoring device can transmit a collected blood oxygen parameter to the central station 14 or the bedside monitor 16. The ECG monitoring device can transmit a collected ECG parameter to the central station 14 or the bedside monitor 16.

It should be understood that the bedside monitor 16 and the central station 14 can achieve data transmission, and control instruction transmission and receiving, through a wireless communicative connection, so as to facilitate remote medical monitoring. In some embodiments, the wireless communicative connection includes WMTS (Wireless Medical Telemetry Services), WLAN (Wireless Local Area Network), Bluetooth, NFC (Near Field Communication), etc., which is not limited.

Since the monitoring system of this disclosure has the aforementioned mobile monitoring device, it also has the advantages of the aforementioned mobile monitoring device.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments arranged at this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, except mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

It should be noted that the above embodiments illustrate this disclosure rather than limit it, and Ordinary technical personnel in this field can design alternative embodiments without departing from the scope of the attached claims.

## Claims

1. A mobile monitoring device, which is fixed to a wearer, which fixation is capable of being unfixed, wherein the mobile monitoring device is configured to collect physiological parameter data of the wearer; wherein the mobile monitoring device is in communicative connection with a target monitoring device, so as to transmit the collected physiological parameter data to the target monitoring device;
**characterized in that**, the mobile monitoring device comprises:
at least one monitoring host, wherein the monitoring host comprises a communication module and a power supply module; wherein the mobile monitoring device is in communicative connection with the target monitoring device through the communication module; and
an accessory part, wherein the monitoring host is coupled to the accessory part, which coupling is capable of being decoupled, and the monitoring host communicates with the accessory part; wherein the accessory part is provided with a first electrical connection part, the monitoring host is provided with a second electrical connection part, the monitoring host and the accessory part communicate with each other through the first electrical connection part and the second electrical connection part;
wherein the accessory part comprises a wearable part and a first memory; the mobile monitoring device is fixed to a designated body part of the wearer through the wearable part, which fixation is capable of being unfixed, the first memory is configured to store first configuration information for performing first configuration on the mobile monitoring device; wherein when the monitoring host is coupled to the accessory part, the monitoring host is configured to obtain, from the first memory, at least a portion of the first configuration information through the second electrical connection part, and perform the first configuration.

2. The mobile monitoring device according to claim 1, **characterized in that**, the first configuration information comprises at least one of: configuration information for wireless communication, wearer information, and configuration information for the monitoring host.

3. The mobile monitoring device according to claim 2, **characterized in that**, the configuration information for wireless communication comprises at least one of: a device identification code, a device serial number code, a randomly generated pairing code, a frequency, a communication channel, a baud rate, an apparatus identification number, an MAC address, an IP address, a service set identifier, a WEP protocol, a WPA protocol, a WPA2 protocol, a WPA3 protocol, an EAP protocol, and a communication port;
the wearer information comprises at least one of: a gender, a classification, an age, a height, a weight, a bed number, a department, and a medical history;
the configuration information for the monitoring host comprises at least one of: a sensor type, a configuration parameter for analysis algorithm, and a configuration parameter for working mode.

4. The mobile monitoring device according to claim 1, **characterized in that**, further comprising:
a sensor, which is connected with a test site of the wearer so as to collect the physiological parameter data;
a parameter cable, wherein one end of the parameter cable is connected with the sensor, and the other end of the parameter cable is connected with the accessory part, so as to transmit to the accessory part the physiological parameter data which is collected by the sensor.

5. The mobile monitoring device according to claim 4, **characterized in that**, the power supply module is in electrical connection with the sensor so as to supply power to the sensor, when the monitoring host is coupled to the accessory part.

6. The mobile monitoring device according to claim 1, **characterized in that**, the power supply module is in electrical connection with the first memory so as to supply power to the first memory, when the monitoring host is coupled to the accessory part.

7. The mobile monitoring device according to claim 1, **characterized in that**, the monitoring host further comprises a second memory, which is configured to store second configuration information for performing second configuration on the mobile monitoring device;
wherein the monitoring host is further configured to obtain, from the second memory, at least a portion of the second configuration information, and perform the second configuration; wherein the second configuration information is at least not completely same as the first configuration information.

8. The mobile monitoring device according to claim 7, **characterized in that**, the second configuration information comprises at least one of: configuration information for wireless communication, wearer information, and configuration information for the monitoring host.

9. The mobile monitoring device according to claim 1, **characterized in that**, further comprising a processor, which is arranged at the monitoring host or the accessory part; wherein the processor is configured to control the communication module to transmit the collected physiological parameter data to the target monitoring device, and/or to process at least a portion of the collected physiological parameter data and control the communication module to transmit a processed result to the target monitoring device.

10. The mobile monitoring device according to claim 9, **characterized in that**, the first configuration at least comprises a pairing operation between the mobile monitoring device and the target monitoring device; the first configuration information comprises configuration information for wireless communication, which information is required to perform the pairing operation between the mobile monitoring device and the target monitoring device;
wherein the processor is further configured to:
perform the pairing operation between the mobile monitoring device and the target monitoring device, while the mobile monitoring device establishes a communicative connection with the target monitoring device for the first time; and
establish the communicative connection between the mobile monitoring device and the target monitoring device, obtain the first configuration information, and output the first configuration information to the first memory for storing, after the mobile monitoring device and the target monitoring device have successfully paired with each other.

11. The mobile monitoring device according to claim 10, **characterized in that**, further comprising a contactless communication unit, which is arranged at the monitoring host or the accessory part; wherein the contactless communication unit is configured to perform contactless communication, so as to read the first configuration information from the target monitoring device or transmit the first configuration information from the mobile monitoring device to the target monitoring device, in a contactless manner.

12. The mobile monitoring device according to claim 10, **characterized in that**, the contactless communication unit comprises at least one of: a near-field communication module, a barcode module, a QR code module, a passive/active radio frequency identification module, an optical module, an infrared module, an ultra-wideband module, or a magnetic pairing module.

13. The mobile monitoring device according to any one of claims 1-11, **characterized in that**, the communication module comprises at least one of: a Bluetooth module, a WMTS communication module, a contactless communication module and a WI-FI communication module.

14. The mobile monitoring device according to any one of claims 1-12, **characterized in that**, the mobile monitoring device further comprises a display which is arranged at the monitoring host and configured to display various visual information.

15. The mobile monitoring device according to any one of claims 1-14, **characterized in that**, the first memory is capable of being arranged at any position of the accessory part.

16. A method for collecting and transmitting physiological parameter data of a wearer, which method is applicable to a mobile monitoring device, **characterized in that**, the method comprises:
collecting the physiological parameter data of the wearer through a sensor which is connected with a test site of the wearer;
transmitting the collected physiological parameter data to an accessory part which is connected with the sensor;
transmitting, through the accessory part, the collected physiological parameter data to a monitoring host; wherein the monitoring host is coupled to the accessory part, which coupling is capable of being decoupled, and the monitoring host communicates with the accessory part, the accessory part comprises a wearable part which is fixed to a designated body part of the wearer, which fixation is capable of being unfixed; and
transmitting, through the monitoring host, the collected physiological parameter data to a target monitoring device; and/or processing at least a portion of the collected physiological parameter data and transmitting a processed result to a target monitoring device;
wherein the method further comprises:
obtaining, by the monitoring host, first configuration information for configuring the mobile monitoring device, from a first memory which is arranged at the accessory part, when the monitoring host is coupled to the accessory part.

17. The method according to claim 16, **characterized in that**, the first configuration information comprises at least one of: configuration information for wireless communication, wearer information, and configuration information for the monitoring host.

18. The method according to claim 17, **characterized in that**, the configuration information for wireless communication comprises at least one of: a device identification code, a device serial number code, a randomly generated pairing code, a frequency, a communication channel, a baud rate, an apparatus identification number, an MAC address, an IP address, a service set identifier, a WEP protocol, a WPA protocol, a WPA2 protocol, a WPA3 protocol, an EAP protocol, and a communication port;
the wearer information comprises at least one of: a gender, a classification, an age, a height, a weight, a bed number, a department, and a medical history;
the configuration information for the monitoring host comprises at least one of: a sensor type, a configuration parameter for analysis algorithm, and a configuration parameter for working mode.

19. The method according to claim 16, **characterized in that**, further comprising:
supplying power to the sensor through a power supply module which is arranged at the monitoring host, when the monitoring host is coupled to the accessory part.

20. The method according to claim 16, **characterized in that**, further comprising:
supplying power to the first memory through a power supply module which is arranged at the monitoring host, when the monitoring host is coupled to the accessory part.

21. The method according to claim 16, **characterized in that**, further comprising:
obtaining second configuration information from a second memory which is arranged at the monitoring host, and performing second configuration on the monitoring host; wherein the second configuration information is at least not completely same as the first configuration information.

22. The method according to claim 21, **characterized in that**, the second configuration information comprises at least one of: configuration information for wireless communication, wearer information, and configuration information for the monitoring host.

23. The method according to claim 16, **characterized in that**, further comprising:
performing a pairing operation between the mobile monitoring device and the target monitoring device, while the mobile monitoring device establishes a communicative connection with the target monitoring device for the first time;
establishing the communicative connection between the mobile monitoring device and the target monitoring device, obtaining the first configuration information, and outputting the first configuration information to the first memory for storing, after the mobile monitoring device and the target monitoring device have successfully paired with each other;
the first configuration at least comprises the pairing operation between the mobile monitoring device and the target monitoring device; the first configuration information comprises configuration information for wireless communication, which information is required to perform the pairing operation between the mobile monitoring device and the target monitoring device.

24. The method according to claim 23, **characterized in that**, further comprising:
performing contactless communication through a contactless communication unit, which is arranged at the monitoring host, so as to read the first configuration information from the target monitoring device or transmit the first configuration information from the mobile monitoring device to the target monitoring device, in a contactless manner.

25. The method according to claim 24, **characterized in that**, the contactless communication unit comprises at least one of: a near-field communication module, a barcode module, a QR code module, a passive/active radio frequency identification module, an optical module, an infrared module, an ultra-wideband module, or a magnetic pairing module.

26. A method for collecting and transmitting physiological parameter data of a wearer, which method is applicable to a mobile monitoring device; **characterized in that**, the mobile monitoring device is fixed to the wearer, which fixation is capable of being unfixed; wherein the mobile monitoring device is configured to collect physiological parameter data of the wearer; wherein the mobile monitoring device is in communicative connection with a target monitoring device, so as to transmit the collected physiological parameter data to the target monitoring device; wherein the mobile monitoring device comprises a monitoring host and an accessory part; the monitoring host is coupled to the accessory part, which coupling is capable of being decoupled, and the monitoring host communicates with the accessory part; the accessory part comprises a wearable part and a first memory; the mobile monitoring device is fixed to a designated body part of the wearer through the wearable part, which fixation is capable of being unfixed; wherein the mobile monitoring device comprises a first monitoring host and a second monitoring host, wherein the method comprises:
obtaining configuration information for wireless communication, which information is required for performing a pairing operation between the first monitoring host and the target monitoring device, when the first monitoring host is coupled to the accessory part;
performing the pairing operation between the first monitoring host and the target monitoring device, based on the configuration information for wireless communication;
establishing a first wireless communicative connection between the first monitoring host and the target monitoring device through a wireless communication module of the first monitoring host, after the first monitoring host and the target monitoring device have successfully paired with each other;
outputting to the first memory for storing first configuration information which comprises the configuration information for wireless communication;
controlling the second monitoring host to obtain, from the first memory, the configuration information for wireless communication, when the first monitoring host is decoupled from the accessory part and the second monitoring host is coupled to the accessory part;
performing a pairing operation between the second monitoring host and the target monitoring device, based on the configuration information for wireless communication; and
establishing a second wireless communicative connection between the second monitoring host and the target monitoring device through a wireless communication module of the second monitoring host, after the second monitoring host and the target monitoring device have successfully paired with each other.

27. The method according to claim 26, **characterized in that**, the first configuration information further comprises at least one of: wearer information and configuration information for the monitoring host.

28. The method according to claim 27, **characterized in that**, the configuration information for wireless communication comprises at least one of: a device identification code, a device serial number code, a randomly generated pairing code, a frequency, a communication channel, a baud rate, an apparatus identification number, an MAC address, an IP address, a service set identifier, a WEP protocol, a WPA protocol, a WPA2 protocol, a WPA3 protocol, an EAP protocol, and a communication port;
the wearer information comprises at least one of: a gender, a classification, an age, a height, a weight, a bed number, a department, and a medical history;
the configuration information for the monitoring host comprises at least one of: a sensor type, a configuration parameter for analysis algorithm, and a configuration parameter for working mode.

29. The method according to claim 26, **characterized in that**, further comprising:
supplying power to the sensor through a power supply module which is arranged at the monitoring host, when the monitoring host is coupled to the accessory part.

30. The method according to claim 26, **characterized in that**, further comprising:
supplying power to the first memory through a power supply module which is arranged at the monitoring host, when the monitoring host is coupled to the accessory part.

31. The method according to claim 26, **characterized in that**, further comprising:
obtaining second configuration information from a second memory which is arranged at the monitoring host, and performing second configuration on the monitoring host; wherein the second configuration information is at least not completely same as the first configuration information.

32. The method according to claim 31, **characterized in that**, the second configuration information comprises at least one of: configuration information for wireless communication, wearer information, and configuration information for the monitoring host.

33. The method according to claim 26, **characterized in that**, further comprising:
performing a pairing operation between the mobile monitoring device and the target monitoring device, while the mobile monitoring device establishes a communicative connection with the target monitoring device for the first time;
establishing the communicative connection between the mobile monitoring device and the target monitoring device, obtaining the first configuration information, and outputting the first configuration information to the first memory for storing, after the mobile monitoring device and the target monitoring device have successfully paired with each other;
the first configuration at least comprises the pairing operation between the mobile monitoring device and the target monitoring device; the first configuration information comprises configuration information for wireless communication, which information is required to perform the pairing operation between the mobile monitoring device and the target monitoring device.

34. The method according to claim 33, **characterized in that**, further comprising:
performing contactless communication through a contactless communication unit, which is arranged at the first monitoring host, so as to read the first configuration information from the target monitoring device or transmit the first configuration information from the mobile monitoring device to the target monitoring device, in a contactless manner.

35. The method according to claim 34, **characterized in that**, the contactless communication unit comprises at least one of: a near-field communication module, a barcode module, a QR code module, a passive/active radio frequency identification module, an optical module, an infrared module, an ultra-wideband module, or a magnetic pairing module.

36. The method according to claim 26, **characterized in that**, obtaining configuration information for wireless communication, which information is required for performing a pairing operation between the first monitoring host and the target monitoring device, when the first monitoring host is coupled to the accessory part, comprises:
obtaining the configuration information for wireless communication from the target monitoring device through a contactless communication unit in a contactless manner; or
obtaining the configuration information for wireless communication from the first monitoring host, and transmitting the configuration information for wireless communication to the target monitoring device through a contactless communication unit in a contactless manner.

37. The method according to claim 27, **characterized in that**, controlling the second monitoring host to obtain, from the first memory, the configuration information for wireless communication, when the first monitoring host is decoupled from the accessory part and the second monitoring host is coupled to the accessory part, comprises:
obtaining the configuration information for wireless communication from an electrical connection part which is arranged at the accessory part, through an electrical connection part which is arranged at the second monitoring host.

38. The method according to claim 26, **characterized in that**, establishing a first wireless communicative connection between the first monitoring host and the target monitoring device through a wireless communication module of the first monitoring host, after the first monitoring host and the target monitoring device have successfully paired with each other, comprises:
broadcasting the configuration information for wireless communication through the wireless communication module of the first monitoring host, so as to enable a wireless communication module of the target monitoring device to establish the first wireless communication connection with the first monitoring host, when the configuration information for wireless communication is detected by the wireless communication module of the target monitoring device; or
detecting the configuration information for wireless communication through the wireless communication module of the first monitoring host; and establishing the first wireless communication connection with the target monitoring device through the wireless communication module of the first monitoring host, when the configuration information for wireless communication, which information is broadcasted through a wireless communication module of the target monitoring device, is detected.

39. The method according to claim 26, **characterized in that**, establishing a second wireless communicative connection between the second monitoring host and the target monitoring device through a wireless communication module of the second monitoring host, after the second monitoring host and the target monitoring device have successfully paired with each other, comprises:
broadcasting the configuration information for wireless communication through the wireless communication module of the second monitoring host, so as to enable a wireless communication module of the target monitoring device to establish the second wireless communication connection with the second monitoring host, when the configuration information for wireless communication is detected by the wireless communication module of the target monitoring device; or
detecting the configuration information for wireless communication through the wireless communication module of the second monitoring host; and establishing the second wireless communication connection with the target monitoring device through the wireless communication module of the second monitoring host, when the configuration information for wireless communication, which information is broadcasted through a wireless communication module of the target monitoring device, is detected.

40. A mobile monitoring device, **characterized in that**, comprising:
a monitoring host, which comprises a power supply module, wherein the power supply module is assembled at the monitoring host, which assembly is capable of being disassembled;
an accessory part, wherein the monitoring host is coupled to the accessory part, which coupling is capable of being decoupled, and the monitoring host communicates with the accessory part; wherein the accessory part is provided with a first electrical connection part, the monitoring host is provided with a second electrical connection part, the monitoring host and the accessory part communicate with each other through the first electrical connection part and the second electrical connection part; wherein the accessory part comprises a wearable part, a sensor, and a first memory; the mobile monitoring device is fixed to a designated body part of the wearer through the wearable part, which fixation is capable of being unfixed, the sensor is connected with a test site of the wearer so as to collect physiological parameter data; the first memory is configured to store first configuration information for performing first configuration on the mobile monitoring device; wherein when the monitoring host is coupled to the accessory part, the monitoring host is configured to obtain, from the first memory, at least a portion of the first configuration information through the second electrical connection part, and perform the first configuration;
a communication module, through which the mobile monitoring device and the target monitoring device are in communicative connection with each other, wherein the communication module is arranged at the monitoring host or the accessory part.

41. A monitoring system, **characterized in that**, comprising:
the mobile monitoring device according to any one of claims 1-15 and 29, wherein the mobile monitoring device is configured to obtain the physiological parameter data, and transmit to the target monitoring device, real-time data and historical data of the physiological parameter data which is obtained by the mobile monitoring device, when the mobile monitoring device is in communicative connection with the target monitoring device;
the target monitoring device, which is in communicative connection with the mobile monitoring device and configured to obtain and display the physiological parameter data.
